# EUROPEAN PATENT SPECIFICATION

(11) **EP 2 043 664 B1**
(45) Date of publication and mention of the grant of the patent: **29.05.2019**
(21) Application number: 07835882.7
(22) Date of filing: 26.06.2007
(51) Int. Cl.: A61K 33/06, A61K 33/14, A61K 33/26, A61K 33/32, A61K 45/06, A61P 1/12

(54) **CALCIUM ALUMINOSILICATE PHARMACEUTICAL**
PHARMAZEUTISCHES CALCIUMALUMINIUMSILIKAT-PRÄPARAT
AGENT PHARMACEUTIQUE À BASE D'ALUMINOSILICATE DE CALCIUM

(30) Priority: 27.06.2006 US 816827 P
(43) Date of publication of application: 08.04.2009
(73) Proprietor: TEXAS ENTEROSORBENTS INC., Bastrop, TX 78602 (US)
(72) Inventor: CARPENTER, Robert, Hunt, Bastrop, TX 78602 (US); HAHN, Kevin, August, Missouri City, TX 77459 (US); KING, Glen, Kenneth, Alvin, TX 77521 (US); ENDICOTT, Melissa, M., Houston, TX 77079 (US)
(74) Representative: Tomkins & Co
(86) International application number: PCT/US2007/014802
(87) International publication number: WO 2008/013630

(56) References cited:
- WO-A-86/06633
- WO-A-2006/067945
- WO-A-2007/011825
- DEMBINSKI Z ET AL: "THE USE OF BENTONITE OF POLISH PRODUCTION IN GASTROINTESTINAL DISEASES OF NEWBORN CALVES" MEDYCYNA WETERYNARYJNA, WARZAW, PL, vol. 41, no. 6, 1 January 1985 (1985-01-01), pages 359-362, XP001539133 ISSN: 0025-8628
- LI S: "Dispersible montmorillonite tablets for treating e.g. acute and chronic diarrhea, gastritis, esophagitis, colitis, inflammation caused by gastrointestinal reflux, and irritable bowel syndrome" WPI / THOMSON,, 23 November 2005 (2005-11-23), XP002453043
- CLARK K J ET AL: "In vitro studies on the use of clay, clay minerals and charcoal to adsorb bovine rotavirus and bovine coronavirus" VETERINARY MICROBIOLOGY, ELSEVIER BV, NL, vol. 63, no. 2-4, 1 October 1998 (1998-10-01), pages 137-146, XP002406623 ISSN: 0378-1135
- BAILEY C ET AL: "Efficacy of montmorillonite clay (NovaSil PLUS) for protecting full-term broilers from aflatoxicosis" JOURNAL OF APPLIED POULTRY RESEARCH, APPLIED POULTRY SCIENCE, ATHENS, GA, US, vol. 15, no. 2, 1 January 2006 (2006-01-01), page 198, XP008095493 ISSN: 1056-6171
- AFRIYIE-GYAWU E ET AL: "Chronic toxicological evaluation of dietary NovaSil clay in Sprague-Dawley rats" FOOD ADDITIVES AND CONTAMINANTS, TAYLOR AND FRANCIS, LONDON, GB, vol. 22, no. 3, 1 January 2005 (2005-01-01), pages 259-269, XP008095565 ISSN: 0265-203X
- WANG J-S ET AL: "Short-term safety evaluation of processed calcium montmorillonite clay ( NovaSil ) in humans" FOOD ADDITIVES AND CONTAMINANTS, TAYLOR AND FRANCIS, LONDON, GB, vol. 22, no. 3, 1 March 2005 (2005-03-01), pages 270-279, XP008095604 ISSN: 0265-203X
- "Product information: NOVASIL PLUS" INTERNET CITATION, [Online] pages 1-2 HOST, XP002493300 Retrieved from the Internet: URL:http://trouwusa.newmind.co.uk/dbimgs/P RODINFORNovaSilPlus.pdf> [retrieved on 2008-08-01]

## Description

### BACKGROUND

This invention is generally related to clay-based compositions. Such compositions may be used in methods for preventing and treating diarrhea. More specifically, the invention relates to an oral composition for use as a supportive therapy for treating diarrhea, possibly associated with treatment of a chemotherapeutic agent in a subject. Other possible causes are radiation, chronic disease, infectious disease, inflammatory proteins, e.g., TNF-α, and treatment with other drugs causing diarrhea in the subject. The composition comprises an effective amount of an isolated calcium aluminosilicate anti-diarrheal ("CASAD"), wherein the isolated CASAD is substantially free from T4-dioxin and toxic heavy metal contamination. The isolated CASAD is capable of binding a chemotherapeutic agent as well as inflammatory proteins and drug metabolites. The compositions can be administered in any suitable form, such as tablet, powder, or suspension form, and can be used as part of any suitable treatment, including oral treatment. Additionally, the clay of this invention does not interfere with the treated systems utilization of important vitamins and micronutrients that are found naturally in the diet. The isolated CASAD of this invention can bind chemotherapeutic agents with high affinity and capacity in the gastrointestinal tract directly, resulting in a notable reduction in gut exposure.

### DIARRHEA

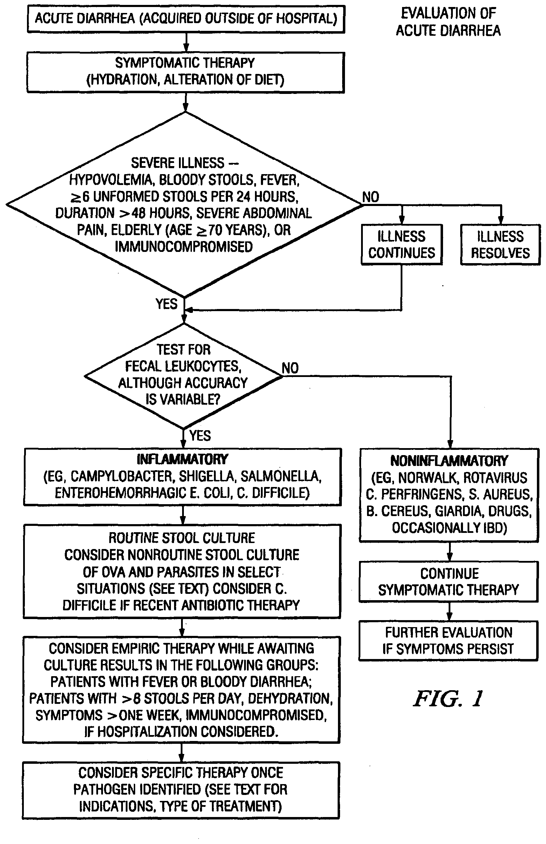

Causes. Diarrheal diseases represent one of the five leading causes of death worldwide, and are a leading cause of childhood death. Morbidity and mortality are significant even in the United States where diarrhea is considered a "nuisance disease" in a normally healthy individual. Diarrhea can be defined as stool weight in excess of 200 grams per day. However, this definition is of little clinical value, since collecting and weighing stools is neither practical nor required except in a clinical research setting. A good working definition is three or more loose or watery stools per day or a definite decrease in consistency and increase in frequency based upon individual baseline. When diarrhea lasts for 14 days it can be considered persistent; the term chronic diarrhea generally refers to diarrhea that lasts for at least one month. Figure 1 shows a flowchart of how acute diarrhea is evaluated.

As a general rule, the principal causes of diarrhea depend upon the socioeconomic status of the population. In developing countries, chronic diarrhea is frequently caused by chronic bacterial, mycobacterial, and parasitic infections, although functional disorders, malabsorption, Crohn's disease, ulcerative colitis, and inflammatory bowel disease are also common. In developed countries, common causes are irritable bowel syndrome (IBS), inflammatory bowel disease, malabsorption syndromes, chronic infections (particularly in patients who are immunocompromised), and patients undergoing chemotherapy, or radiation therapy.

Diarrhea reflects increased water content of the stool, whether due to impaired water absorption and/or active water secretion by the bowel. In severe infectious diarrhea, the number of stools may reach 20 or more per day, with defecation occurring every 20 or 30 minutes. In this situation, the total daily volume of stool may exceed two liters, with resultant volume depletion and hypokalemia. Most patients with acute diarrhea have three to seven movements per day with total stool volume less than one liter per day

The overall burden of acute diarrhea in the United States and other developed countries has not been well-studied. Thus, the economic impact of diarrhea has not been well-quantified, particularly when considering societal costs. One estimate suggests that chronic diarrhea costs more than $350,000,000 annually from work-loss alone (Everhart, JE (Ed). Digestive Disease in the United States: Epidemiology and impact. NIH Publ 94-1447. Bethesda, MD: National Institutes of Health, 1994.) Additionally, chronic diarrhea has been shown to decrease quality of life. However, accurate assessment of the degree to which this occurs has not been established. One explanation is that a well-validated disease-specific quality-of-life instrument has not yet been developed. Furthermore, no studies have attempted to measure quality of life in large groups of patients. Chronic diarrhea was an independent predictor of decreased quality of life in HIV-infected patients.

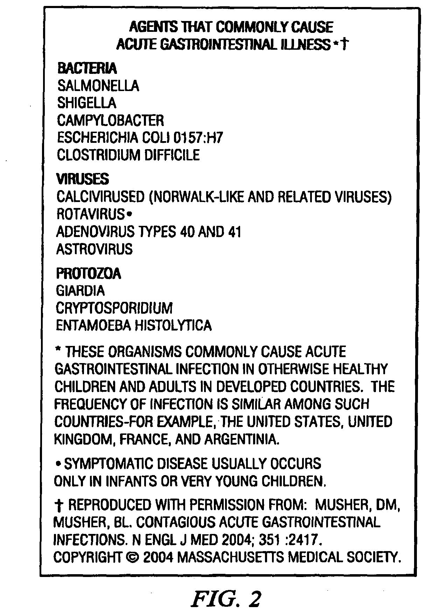

Infectious or noninfectious causes may be responsible for acute diarrhea and, in selected patients, both can occur simultaneously. Noninfectious causes of diarrhea include drugs, food allergies, primary gastrointestinal diseases such as inflammatory bowel disease, and other disease states such as thyrotoxicosis and the carcinoid syndrome. A variety of infectious diseases cause acute diarrhea. Figure 2 shows agents that commonly cause acute gastrointestinal illness.

Generally, most cases of acute diarrhea are self-limiting, whether the cause is an infection, including viruses, or non-infectious. While acute diarrhea occurs in most cases because of food borne illness, there are other causes of acute diarrhea induced by waterborne outbreaks associated with recreational water (e.g., swimming or wading pools). It has been estimated that approximately one-half of these outbreaks involve gastroenteritis. The outbreaks were associated most frequently with Cryptosporidium (50 percent) in treated water sources and with toxigenic Escherichia coli (25 percent) and norovirus (25 percent) in freshwater sources. The frequency of the most common bacterial pathogens were: Campylobacter - 2.33 percent (42 percent of isolates); Salmonella - 1.82 percent (32 percent of isolates); Shigella - 1.06 percent (19 percent of isolates); E. coli O157:H7, the major enterohemorrhagic strain - 0.39 percent overall (7 percent of isolates) but much more common in visibly bloody isolates (7.8 versus 0.14 percent in specimens without visible blood). Yersinia, Listeria, and Vibrio each accounted for less than 1 percent of cases. An important limitation to these data are possible selection bias since only a small proportion of patients seek medical attention and are investigated. Additionally, there are at least four viral agents that are medically important causes of viral gastroenteritis: norovirus (also known as Norwalk-like virus), rotavirus, enteric adenoviruses, and astroviruses.

Parasitic pathogens are also etiologic agents of diarrhea in developed countries. Some parasitic pathogens include cyclospora, giardia lamblia, cryptosporidium, and entamoeba histolytica. One of ordinary skill in the art will recognize that select populations are at greater risk for infection with enteric pathogens. For example, diarrhea is common in an immunocompromised host, and the frequencies can be different in immunocompetent and immunocompromised patients. Individuals with immunocompromising illnesses such as lymphoma, bone marrow transplantation, or human immunodeficiency virus (HIV) infection may be at particular risk. Diarrhea has been reported in up to 60 percent of patients with the acquired immunodeficiency syndrome (AIDS) from developed countries and in as many as 95 percent of patients with AIDS from the developing world. Before AIDS, the most common pathogens included the parasitic organisms Cryptosporidium parvum, Isospora belli, Cyclospora, and Microsporidia, the bacterial pathogens Salmonella enteritidis, Campylobacter, Shigella species, and Mycobacterium avium complex, and the viral pathogens, cytomegalovirus, herpes simplex, and adenovirus.

The frequency with which these organisms have been identified as causes of diarrheal disease in patients with AIDS has been decreasing, presumably related to the use of highly active antiretroviral therapy (HAART), although diarrheal illness remains a common syndrome in these patients.

Nosocomial diarrhea is defined as the new onset of diarrhea at least 72 hours after hospital admission. Comprehensive studies have been limited, but nosocomial diarrhea appears to increase the length of stay in hospitalized adults by an average of more than one week, and by more than one month in the elderly. The incidence and mortality rate are greatest in patients over the age of 70 years.

*C. difficile* is a spore forming bacteria which can be part of the normal intestinal flora in as many as 50% of children under age two, and less frequently in individuals over two years of age. *C. difficile* is the major cause of pseudomembranous colitis and antibiotic associated diarrhea. *C. difficile-associated* disease occurs when the normal intestinal flora is altered, allowing *C. difficile* to flourish in the intestinal tract and produce a toxin that causes a watery diarrhea. Repeated enemas, prolonged nasogastric tube insertion and gastrointestinal tract surgery increase a person's risk of developing the disease. The overuse of antibiotics, especially penicillin (ampicillin), clindamycin and cephalosporins may also alter the normal intestinal flora and increase the risk of developing *C. difficile* diarrhea.

Mild cases of *C. difficile* disease are characterized by frequent, foul smelling, watery stools. More severe symptoms, indicative of pseudomembranous colitis, include diarrhea that contains blood and mucous, and abdominal cramps. An abnormal heart rhythm may also occur.

Chemotherapy-induced diarrhea (CID) occurs in thousands of patients on a yearly basis. CID is described commonly with fluoropyrimidines (particularly 5-fluorouracil [5-FU]), irinotecan, methotrexate, and cisplatin. However, one of ordinary skill in the art will recognize that other chemotherapy agents can cause diarrhea. Diarrhea is often dose-limiting and a source of major toxicity of regimens containing a fluoropyrimidine, irinotecan and/or other chemotheraputic agents.

For example, both 5-FU and irinotecan cause acute damage to the intestinal mucosa, leading to loss of epithelium. Although not wanting to be bound by theory, 5-FU causes mitotic arrest of crypt cells, leading to an increase in the ratio of immature secretory crypt cells to mature villous enterocytes. The increased volume of fluid that leaves the small bowel exceeds the absorptive capacity of the colon, leading to clinically significant diarrhea.

With compounds such as irinotecan, early onset diarrhea occurs during, or within several hours, of drug infusion in 45 to 50 percent of patients and is cholinergically mediated. This effect is thought to be due to structural similarity of the drug with acetylcholine. In contrast, late irinotecan-associated diarrhea is not cholinergically mediated. The pathophysiology of late diarrhea appears to be multifactorial with contributions from dysmotility and secretory factors as well as a direct toxic effect on the intestinal mucosa.

Irinotecan produces mucosal changes associated with apoptosis, such as epithelial vacuolization, and goblet cell hyperplasia, suggestive of mucin hypersecretion. On the other hand, experimental studies have shown that inhibition of intestinal beta-glucuronidase activity with antibiotics may protect against mucosal injury and ameliorate the diarrhea.

The use of anthracyclines (doxorubicin, having the trade name Adriamycin®) can be associated with gastrointestinal problems. Acute nausea and vomiting occurs frequently and may be severe. This may be alleviated by antiemetic therapy. Mucositis (stomatitis and esophagitis) may occur 5 to 10 days after administration. The effect may be severe, leading to ulceration, and represents a site of origin for severe infections. The dosage regimen consisting of administration of doxorubicin on three successive days results in greater incidence and severity of mucositis. Ulceration and necrosis of the colon, especially the cecum, may occur, leading to bleeding or severe infections which can be fatal. This reaction has been reported in patients with acute non-lymphocytic leukemia treated with a 3-day course of doxorubicin combined with cytarabine. Anorexia and diarrhea have also been reported.

Cisplatin has been used for treatment of head and neck, breast, gastric, lung, esophageal, cervical, prostate and small cell lung cancer; Hodgkin's and non-Hodgkin's lymphoma; neuroblastoma; sarcomas, myeloma, melanoma, mesothelioma, and osteosarcoma. The adverse reaction to cisplatin include gastrointestinal nausea, vomiting and diarrhea.

CID can be debilitating and, in some cases, life-threatening. Findings in such patients include volume depletion, renal insufficiency, and electrolyte disorders such as hypokalemia, metabolic acidosis, and, depending upon water intake, hyponatremia (increased water intake that cannot be excreted because of the hypovolemic stimulus to the release of antidiuretic hormone), or hypematremia (insufficient water intake to replace losses). CID can also lead to treatment delays, increased cost of care, reduced quality of life, and diminished compliance with treatment regimens.

Radiation therapy (RT) is a common form of treatment for patients with gynecologic, genitourinary, gastrointestinal, and other cancers. Bowel toxicity, manifested primarily by radiation induced diarrhea (RID), is the most common form of acute toxicity for these patients. Radiation therapy can be used alone or combined with chemotherapy for a one-two punch to the gastrointestinal tract. Radiation induced diarrhea (RID) occurs in about 160,000 patients annually. Without wanting to be bound by theory, RID is likely caused by inflammation of the bowel through the release of inflammatory proteins, or cytokines.

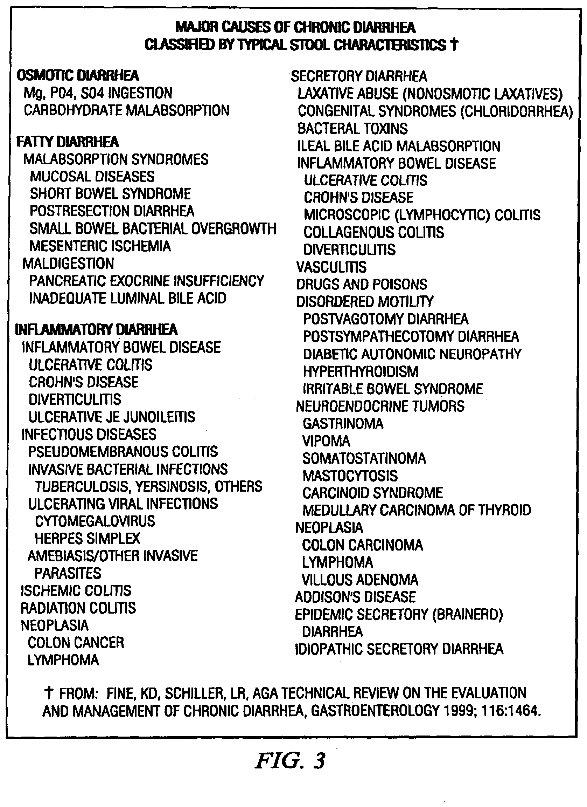

Figure 3 shows major causes of chronic diarrhea classified by typical stool characteristics.

Treatment of Diarrhea. The management of patients with diarrhea begins with general measures such as hydration and alteration of diet. Antibiotic therapy is generally not required in most cases since the illness is usually self-limited. Nevertheless, empiric and specific antibiotic therapy can be considered in certain situations.

The most common therapy in diarrheal illness is hydration, preferably by the oral route with solutions which contain water, salt, and sugar. Oral rehydration therapy is grossly underutilized in the United States where health care providers tend to overuse intravenous hydration. Oral rehydration solutions were developed following the realization that, in many small bowel diarrheal illnesses, intestinal glucose absorption via sodium-glucose co-transport remains intact. Thus, in diarrheal disease caused by any organism which depends on small bowel secretory processes, the intestine remains able to absorb water if glucose and salt are also present to assist in the transport of water from the intestinal lumen.

The World Health Organization oral rehydration solution (per liter of water) (WHO-ORS) composition consists of: about 3.5 g sodium chloride; about 2.9 g trisodium citrate or 2.5 g sodium bicarbonate; about 1.5 g potassium chloride, and about 20 g glucose or 40 g sucrose. The WHO-ORS is available from the manufacturer (Jianas Brothers, St. Louis, Mo). Rehydralyte (Ross Laboratories, Columbus, Ohio) is available over the counter, but contains 20 percent less sodium, so larger volumes are needed for rehydration. A similar solution can be made by adding one-half teaspoon of salt, one-half teaspoon of baking soda, and four tablespoons of sugar to one liter of water. Cera-lyte is also available over the counter and is a rice-based oral rehydration solution.

Bismuth subsalicylate (Pepto-Bismol), 30 mL or two tablets every 30 minutes for eight doses, may be useful in some patients. It seems to be most effective in those in whom vomiting is a prominent feature of their illness. Bismuth subsalicylate has both anti-inflammatory and antibacterial actions but has not been extensively evaluated for the treatment of CID.

Acetorphan is an enkephalinase inhibitor that blocks epithelial cyclic AMP-mediated secretion. It has moderate activity in patients with irinotecan-induced diarrhea. Budesonide is a glucocorticoid that has high affinity for the glucocorticoid receptor but low systemic activity due to extensive first-pass metabolism in the liver. Budesonide is effective for inducing remission in ileal or ileal cecal Crohn's disease.

Probiotics, including bacteria which assist in recolonizing the intestine with non-pathogenic flora and shortening diarrhea, can also be used as alternative therapy. Probiotics have been shown to be useful in treating *C. difficile,* traveler's diarrhea and acute non-specific diarrhea in children.

The treatment of diarrhea in an immunocompromised patients (e.g. one early HIV infection) does not differ from that used in non-immunocompromised hosts. Patients who are more immunocompromised (absolute CD4 count less than 200/µL) should be treated with empiric antimicrobial therapy with a fluoroquinolone for the bacterial enteritis.

The treatment of CID or RID includes non-pharmacologic and pharmacologic interventions to slow the diarrhea and careful serial evaluation to rule out significant volume depletion or comorbidities that would require targeted intervention or hospitalization. Initial nonpharmacologic measures include avoidance of foods that would aggravate the diarrhea and aggressive oral rehydration with fluids that contain water, salt, and sugar (since glucose promotes intestinal sodium absorption) such as broth or GATORADE®. These principles are similar to those used for infectious diarrhea.

Loperamide, an opiate, is a mainstay of therapy for CID. Loperamide (Imodium) and diphenoxylate (Lomotil) are the most commonly used and both are FDA-approved for this indication. Both give a rapid onset of action. Loperamide appears to be more effective and has been recommended in treatment guidelines. The standard dose of loperamide is an initial 4 mg dose followed by 2 mg every four hours or after every formed stool. This regimen is only moderately effective in CID and a more aggressive regimen (4 mg initially, then 2 mg every two hours or 4 mg every four hours until diarrhea-free for 12 hours) is often required, particularly for irinotecan-induced diarrhea.

In another report, irinotecan was given weekly at 125 mg/m² for four weeks with two weeks rest. The prevalence of grade 3/4 diarrhea fell from 56 to 9 percent with strict adherence to the high-dose loperamide regimen.

Octreotide is a synthetic long-acting somatostatin analog that is believed to act via several mechanisms: decreased secretion of a number of hormones, such as vasoactive intestinal peptide (VIP); prolongation of intestinal transit time; and reduced secretion and increased absorption of fluid and electrolytes. Octreotide is approved by the Food and Drug Administration for the treatment of diarrhea related to VIP-secreting tumors and symptoms due to carcinoid syndrome.

Octreotide is also beneficial in patients with CID from fluoropyrimidines and irinotecan, although the optimal dose has not been determined. Although one randomized trial in 41 5-FU-treated patients showed that octreotide was more effective than standard-dose loperamide (90 versus 15 percent resolution of diarrhea by day three), octreotide is generally reserved as a second-line therapy for patients who do not respond to high dose loperamide because of its high cost and the general effectiveness of loperamide,.

The recommended starting dose of octreotide is 100 to 150 µg subcutaneously, three times a day. However, several reports suggest that higher doses (500 µg) may be more effective. The available data support upward titration of the dose (up to 2500 µg three times daily) in nonresponders. The side effects of octreotide are generally mild, including bloating, cramping, flatulence and fat malabsorption. Hypersensitivity-like reactions and hypoglycemia can occur at higher doses.

Other antidiarrheal agents have been used with patients having CID, but are not common. For example, Anticholinergic drugs are not commonly used because of side effects. However, they can be helpful when diarrhea is associated with significant cramping. Absorbents (e.g., pectin, aluminum hydroxide) and adsorbents (e.g., kaolin, charcoal) bind osmotically active substances and can be effective adjunctive therapy in patients with mild diarrhea. Deodorized tincture of opium (DTO), is a widely used antidiarrheal agent, despite the absence of literature reports supporting efficacy for treatment of chemotherapy-induced diarrhea. DTO contains the equivalent of 10 mg/mL morphine. The recommended dose is 10 to 15 drops in water every 3 to 4 hours. An alternative is paregoric, camphorated tincture of opium, a less concentrated preparation that contains the equivalent of 0.4 mg/mL morphine. The recommended dose is 5 mL (one teaspoonful) in water every 3 to 4 hours.

Although there is a well-recognized risk of diarrhea with certain chemotherapy regimens (eg, irinotecan, 5-FU with high dose leucovorin administered as a five day bolus once monthly), few studies have investigated the potential benefit of prophylactic antidiarrheal therapy.

Activated charcoal may have a role in preventing irinotecan-induced diarrhea. In one study, 28 patients received activated charcoal during the first treatment cycle, but not the second. The incidence of grade 3 or 4 diarrhea increased from 7 to 25 percent between cycles 1 and 2, and more patients required 10 or more tablets of loperamide without prophylaxis. Activated charcoal may also absorb beneficial nutrients, which is a clear disadvantage of its use.

At present, prophylactic antidiarrheal treatment is not a standard approach for any regimen. Oral activated charcoal may be considered in patients treated with irinotecan.

### CLAY AS A TREATMENT FOR DIARRHEA.

The clay-based composition of this invention, also referred to as CASAD, can be used to treat and prevent inflammation induced by chemicals, viral co-carcinogenesis, and cytokines, and/or to treat or prevent diarrhea. However, one of ordinary skill in the art will recognize that there are many different types of clay, and clay has a very long history in human medical history.

Clay is a generic term for an aggregate of hydrous silicate particles. Generally, clay consists of a variety of phyllosilicate minerals generally rich in silicon and aluminum oxides, and hydroxides. Clays are distinguished from other small particles present in soils, such as silt, by their small size, flake or layered shape, affinity for water and high plasticity index. Main groups of phyllosilicate clays include kaolinite, montmorillonite-smectite, illite, and chlorite.

Montmorillonite clay is typically formed as a weathering product of low silica rocks. Montmorillonite is a member of the smectite group and a major component of bentonite.

Varve (or varved clay) is clay with visible annual layers, formed by seasonal differences in erosion and organic content. This type of deposit is common in former glacial lakes from the ice age.

Quick clay is a unique type of marine clay, indigenous to the glaciated terrains of Norway, Canada, and Sweden. It is a highly sensitive clay, prone to liquefaction which has been involved in several deadly landslides.

Other names for clay include: HSCAS, Akipula, aluminium silicate, anhydrous aluminum silicates, askipula, beidellitic montmorillonite, benditos, bioelectrical minerals, cipula, chalk, clay dirt, clay dust, clay lozenges, clay suspension products, clay tablets, colloidal minerals, colloidal trace minerals, fossil farina, humic shale, Indian healing clay, kaolin, kipula, mountain meal, panito del senor, plant-derived liquid minerals, tirra santa, Terra sigillata, white clay, white mud, etc.

Today, clay is used in many industrial processes to make bricks, cooking pots, art objects, dishware, sparkplug bodies, cement production and chemical filters. According to folklore, eating clay has many medicinal purposes, but the scientific literature indicates that ingesting certain clays may be harmful to the consumer. The chemical nature of clays may allow them to absorb a variety of potentially detrimental agents. For example, clay pots containing candy (Jarritos brand Tamarindo candy) have been recalled in the United States by the Food and Drug Administration due to high levels of lead in the candy that was derived from the clay pots. Clay products may contain varying amounts of aluminum, arsenic, barium, lead, nickel, titanium and other trace metals. Additionally, elevated levels of 2,3,7,8-tetracholorodibenzo-p-dioxin have been found in farm-raised catfish and eggs from chickens fed a diet including ball clay from a mine in Mississippi. Additionally, chronic clay eating may be associated with trace element deficiency. However, it should be pointed out that the group of clays used predominantly in the ceramics industry and consumed by humans are the kaolinites (Ball clays).

Therefore, clays (especially kaolinites) that are ingested by humans should not have elevated levels of toxic agents. The clay-based composition of this invention can be used to treat or prevent aflatoxin toxicity. Although clay has been used medicinally for centuries in Africa, India, and China, and by Native American groups, one of ordinary skill in the art understands there is a potential for severe adverse effects with chronic oral ingestion of certain clays. As described below, the scientific and medical communities believe these adverse effects may outweigh any potential benefits.

The practice of eating dirt, clay, or other non-nutritious substances may be referred to as "pica" or "geophagia," and is common in early childhood and in mentally handicapped or psychotic patients. There is some evidence that mineral deficiencies such as iron deficiency may lead to pica, and prevalence is higher in developing countries and in poor communities. Chronic clay ingestion may lead to iron malabsorption and further precipitate this condition. There is insufficient scientific evidence to recommend for or against the use of clay for any medical condition. The potential for adverse effects with chronic oral ingestion of clay may outweigh any potential benefits.

Clay products may contain varying amounts of aluminum, arsenic, barium, lead, nickel, titanium and other trace metals. Certain colloidal mineral supplements may also contain unsafe concentrations of radioactive metals. Ingestion of certain clays is possibly unsafe when used in patients during pregnancy or lactation, or when used in children. Some clays may possess potassium-binding capacity, and chronic ingestion of these clays has been associated with severe hypokalemia, particularly in patients with renal insufficiency. It has been suggested that habitual eating of kaolinic clays (pica or geophagia) may lead to iron malabsorption and severe deficiency, and may be associated with anemia and lead poisoning.

The following physiological problems have been reported with "pica" or "geophagia:"
Allergy/hypersensitivity to certain clay, can be characterized by an edematous appearance, dilated cardiomyopathy, polyuria, and death. Additionally, skin dryness, skin ulcerations were noted over the upper and lower extremities of subjects.
Neurologic/CNS: Pica has been associated with the development of lead poisoning in children, and may carry a risk of central nervous system damage. In one case report, a 6-year-old girl died from complications of lead poisoning and encephalopathy after ingesting lemonade from a glazed clay pitcher. The risk of neurolathyrism, a neurodegenerative, irreversible disorder that cause spastic paraparesis of the body that leads to paralysis, was reported to quadruple in a case-control study in Ethiopia when cooking grass pea with clay utensils.
Psychiatric: Habitual pica may occur in patients with mental illness, including psychotic disorders.
Pulmonary/Respiratory: In the 1960s, it was reported that children with a history of pica were predisposed to develop more frequent and severe respiratory infections than healthy children. Chronic bronchitis, dyspnea, and pneumoconiosis have been associated with dust exposure in the heavy clay industry.
Cardiovascular: Pica was reported to be associated with dilated cardiomyopathy and death.
Gastrointestinal: Clay eating may precipitate constipation or diarrhea. Heartburn, flatulence, loss of appetite, and vomiting after meals have also been reported. Clay eating has also been associated with intestinal obstruction and necrotizing enteritis, leading to bowel perforation. Colonic stones have been reported in two children with pica. Geophagia has been associated with hepatosplenomegaly.
Renal: Clay possesses potassium-binding capacity, and chronic clay ingestion has been associated with severe hypokalemia, particularly in patients with renal insufficiency, but not in those receiving hemodialysis.
Endocrine: Myopathy due to severe hypokalemia has been reported in 1 case report with large quantities of clay ingestion.
Genitourinary: Chronic clay eating has been associated with polyuria and urge incontinence, as well as hypogonadism.
Hematologic: Pica may lead to iron malabsorption and severe deficiency, and has been associated with anemia.
Musculoskeletal: Myositis has been associated with chronic clay ingestion. Myopathy due to severe hypokalemia has been reported with large quantities of clay ingestion.
Infectious Disease: Hookworm infections have been associated with ingestion of clay. Tetanus contracted from clay has been described in an infant who ate clay, and in a newborn whose umbilical cord was wrapped in clay.
Iron, Calcium. Magnesium: Certain clay may act as a cation exchange resin. Calcium and magnesium in these clays can be exchanged with iron, making iron unavailable because of formation of insoluble iron complexes. Iron deficiency may result, and levels of calcium or magnesium may increase.
Potassium: Certain clays possess potassium-binding capacity, and have been associated with hypokalemia.

One of ordinary skill in the art understands that there is insufficient scientific and clinical evidence in the literature to recommend for or against the medicinal use of certain clays, however, the current illustrations in medicine tend to teach away from using clay as a safe treatment in patients with aflatoxin poisoning, or liver cancer in predisposed systems, including human systems. The methods and compositions of this invention utilize isolated clay compositions that are not typically consumed by humans or used in the manufacture of ceramic eating and drinking utensils. The processed clay of this invention has a particular chemical makeup that does NOT impart adverse health effects when administered orally (based on extensive scientific studies in humans and animals).

Dembinski et al (Medycyna Weterynaryjna, Warzaw, PL, vol. 41, no. 6, page 359-362) describe the use of a bentonite of Polish origin in gastrointestinal diseases of newborn calves.

Li ("Dispersible montmorillonite tablets for treating e.g. acute and chronic diarrhea, gastritis, esophagitis, colitis, inflammation caused by gastrointestinal redlux and irritable bowel syndrome") describes dispersible montomillonite tablets for treating diarrhea caused by gastrointestinal reflux and irritable bowel syndrome. The tablets comprise filler, disintegrant and smectite.

Clark et al (Veterinary Microbiology, 63 (1998) 137) describes in vitro studies on the use of clay, clay minerals and charcoal to adsorb bovine rotavirus and bovine coronavirus.

WO8606633 describes an anti-diarrhea composition comprising a hydrous magnesium aluminium silicate clay, a sodium salt, a potassium salt and a sugar.

Bailey et al, "Efficacy of Montmorillonite clay (NovaSil PLUS) for protecting full-term broilers from aflatoxicosis' Journal of Applied Poultry Research, Applied Poultry Science, 15 (1 January 2006) page 198 discloses the use of Montmorillonite clay fed as NovaSil PLUS in the protection of birds against the effects of aflatoxin.

In one aspect the present invention provides an oral composition for use as a supportive therapy for treating diarrhea in a subject as defined in the claims. A first aspect of the invention is a composition for use as a supportive therapy for treating and preventing diarrhea. The diarrhea may be associated with chronic or infectious disease, treatment using a chemotherapeutic agent or radiation therapy in a subject, or treatment involving any drug that might cause diarrhea. The composition comprises an effective amount of an isolated calcium aluminosilicate anti-diarrheal ("CASAD") clay, wherein the isolated CASAD has a low sodium content, is substantially free from T4-dioxin and toxic heavy metal contamination, and is capable of treating diarrhea. The clay can be in any suitable form, preferably in tablet, powder, or suspension form, and can be administered according to any suitable administration protocol, including orally. The isolated low-sodium, calcium aluminosilicate clay has a chemical composition comprising: CaO above about 3.2%; MgO about 4.0 - 5.4%; Fe₂O₃ about 5.4 - 6.5; K₂O about 0.50 - 0.90%; Na₂O about 0.10 - 0.30%; MnO about 0.01 - 0.03%; Al₂O₃ about 14.8 - 18.2%; and SiO₂ about 62.4 - 73.5%, wherein the chemical composition is determined by x-ray fractionation as a weight percent. The calcium aluminosilicate clay has an average particle size that is between about 5 and 100 microns, preferably less than about 80 microns, and most preferably less than about 50 microns, and is in the pH range of about 5 to 9 in a suspension or solution. The CASAD is capable of binding chemotheraputic agents such as doxorubicin, inflammatory proteins, and drug metabolites. The present invention also provides for use of an effective amount of an isolated low sodium CASAD clay in the preparation of a medicament for supportive therapy of symptoms of diarrhea as described in the claims.

The diarrhea may be associated with chronic or infectious disease, any disease or condition causing inflammation, or treatment of the subject with a drug or chemotherapeutic agent or radiation therapy. The composition for use comprises an effective amount of an isolated calcium aluminosilicate anti-diarrheal ("CASAD") clay, wherein the isolated CASAD has a low sodium content, is substantially free from T4-dioxin and toxic heavy metal contamination, and is capable of treating diarrhea. The CASAD can be in any appropriate form, particularly tablet, powder, capsule, or suspension forms, and can be administered orally or by any commonly known administration methods. In a particular example, the current invention pertains to a method of treating diarrhea in animals and humans, including children.

Another aspect of the current disclosure is a method of creating a CASAD clay formulation comprising the step of sizing the clay particles so that they are between about 5 and 100 microns, preferably less than 80 microns, and most preferably less than 50 microns. An additional aspect of the current invention is a method of creating a CASAD clay formulation that comprises additional ingredients, namely, additional drugs or pharmaceutical agents or inert ingredients such as pharmaceutically acceptable carriers. The method comprises the steps of mixing the CASAD clay with the one or more additional ingredients.

Also disclosed is a method of mitigating an effect of a cytokine (e.g. TNF-α) in persons predisposed to systemic inflammation and acute phase responses (e.g. various autoimmune disorders, rheumatoid arthritis, Crohn's disease, or psoriasis). The method comprises: (a) administering orally an effective amount of an isolated calcium aluminosilicate ("CAS") clay, wherein the isolated CAS is substantially free from T4-dioxin and toxic heavy metal contamination, and is capable of binding the environmental toxin (e.g. aflatoxin); (b) waiting a period of time (e.g. less than about 24 hours); and (c) repeating step (a)-(b) until the effects of the effects of cytokines are mitigated. The isolated CAS has a chemical composition comprising: CaO above 3.2%; MgO about 4.0 - 5.4%; Fe₂O₃ about 5.4 -6.5; K₂O about 0.50 - 0.90%; Na₂O about 0.10 - 0.30%; MnO about 0.01 - 0.03%; Al₂O₃ about 14.8 - 18.2%; and SiO₂ about 62.4 - 73.5%; wherein the chemical composition is determined by x-ray fractionation as a weight percent. The isolated CAS has an average particle size that is between about 5 and 100 microns, preferably less than about 80 microns, and most preferably less than about 50 microns, and is in the pH range of about 5 to 9 in suspension or solution.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.
FIGURE 1 shows a flowchart of how acute diarrhea is evaluated.
FIGURE 2 shows agents that commonly cause acute gastrointestinal illness.
FIGURE 3 shows major causes of chronic Diarrhea classified by typical stool characteristics.
FIGURE 4 shows data representing mass spectrometry derived analytical data that show the absorptive capacity of CASAD for the cancer chemotherapeutic agent doxorubicin (also known as Adriamycin). The two bottom spectra represent analyses of a doxorubicin solution (1000 ng/ml) while the upper two chromatograms and the near absence of drug in solution after addition of 1 mg/ml CASAD (upper two chromatograms).
FIGURE 5 shows a table indicating toxic compounds that can be bound by the CASAD.
FIGURE 6 shows two graphs indicating that CASAD can bind TNF-α.
FIGURE 7 shows how clay was selected for testing due to its GRAS status and its purity including priority trace metals and dioxin levels.
FIGURE 8 shows the classification of isotherms by shape.
FIGURE 9 shows the isotherms of regular vs. collapsed HSCAS.
FIGURE 10 shows the results of a ¹³C-aminopyrine demethylation blood test in 16 dogs before and after undergoing chemotherapy with doxorubicin.
FIGURE 11 shows the measure of fecal alpha(1)-proteinase inhibitor (α-1PI) before and following adriamycin administration.

### DETAILED DESCRIPTION

Before describing the present invention in detail, it is to be understood that this invention is not limited to particular compositions or composition delivery systems, which may vary. It is also to be understood that the terminology used herein is for the purpose of describing particular embodiments only, and is not intended to be limiting. In addition, before describing detailed embodiments of the invention, it will be useful to set forth definitions that are used in describing the invention. The definitions set forth apply only to the terms as they are used in this patent and may not be applicable to the same terms as used elsewhere, for example in scientific literature or other patents or applications including other applications by these inventors or assigned to common owners. Additionally, when examples are given, they are intended to be exemplary only and not to be restrictive.

It must be noted that, as used in this specification and the appended claims, the singular forms "a," "an" and "the" include plural referents unless the context clearly dictates otherwise. Thus, for example, reference to "a pharmacologically active agent" includes a mixture of two or more such compounds, reference to "a base" includes mixtures of two or more bases, and the like.

In describing and claiming the present invention, the following terminology will be used in accordance with the definitions set out below.

"Active agent," "pharmacologically active agent," "pharmaceutical agent," "composition," and "drug" are used interchangeably herein to refer to compositions and drugs that are useful for the prevention and treatment of chemical viral co-carcinogenesis, aflatoxin induced liver cancer, cytokine induced inflammation, or diarrhea. The terms also encompass pharmaceutically acceptable, pharmacologically active derivatives and analogs of such drugs, including, but not limited to, salts, esters, amides, prodrugs, active metabolites, inclusion complexes, analogs, and the like. Therefore, when the terms "active agent," "pharmacologically active agent", or "drug" are used, it is to be understood that applicants intend to include the active composition per se as well as pharmaceutically acceptable, pharmacologically active salts, esters, amides, pro-drugs, active metabolites, inclusion complexes, analogs, etc., which are collectively referred to herein as "pharmaceutically acceptable derivatives."

The present invention pertains to compositions and methods of preventing or treating diarrhea using an effective amount of a clay-based composition, also known as CASAD. Diarrhea is a common sign of toxicity of both cytotoxic chemotherapy and radiation therapy. Standard of care for diarrhea is rehydration and the administration of antibiotics, motility modifiers and anti-secretory agents. While these treatments are useful in the acute setting, some humans and companion animals will fail to improve or may require long term therapy. Adverse effects of these medications in both the acute and chronic setting can be problematic for the human patient or to the companion animal, owner and clinician.

The clay-based compositions of the present invention can be administered by any suitable route, for example by oral, topical, buccal, inhalation, sublingual, rectal, vaginal, transurethral, nasal, topical, percutaneous, i.e., transdermal, or parenteral (including intravenous, intramuscular, subcutaneous, and intracoronary) administration. Parenteral administration can be accomplished using a needle and syringe, or infused together with an IV fluid, like 5% dextrose or normal saline.

Other pharmaceutical agents that can be used in association with the clay-based composition include drugs acting at synaptic and neuroeffector junctional sites, drugs acting on the central nervous system, an autacoid, drugs that treat inflammation, cardiovascular drugs, drugs affecting renal function and electrolyte metabolism, drugs affecting uterine motility, locally acting drugs, drugs for parasitic diseases, drugs for microbial diseases, drugs for neoplastic diseases, drugs acting on the blood and the blood-forming organs, hormones, hormone antagonists, or vitamins. Examples of a pharmaceutical agents include, but are not limited to, an antibiotic, a chemotherapeutic agent, an anti-diarrhea agent, a steroid, an opioid, and a gastric antacid.

Although not wanting to be bound by theory, no absolute methods are available for totally eliminating diarrhea; however, clay-based approaches do offer a economical and practical solution for reducing dietary symptoms of diarrhea. Furthermore, the use of dietary enterosorbents and nonspecific binding agents to prevent and treat the symptoms of diarrhea are described in the examples below.

### EXAMPLES

The following examples are provided to further illustrate this invention and the manner in which it may be carried out. It will be understood, however, that the specific details given in the examples have been chosen for purposes of illustration only and not be construed as limiting the invention.

### EXAMPLE 1

GRAS Status and Safety Studies for *in vivo* Use of Clay. One of ordinary skill in the art would be aware that scientific publications support the use of calcium montmorillonite clay in animal feeds. For example, hydrated sodium calcium aluminosilicate (HSCAS) is generally recognized as safe for use in feeds at a level not exceeding 2.0% (w/w) in accordance with good manufacturing or feeding practice.

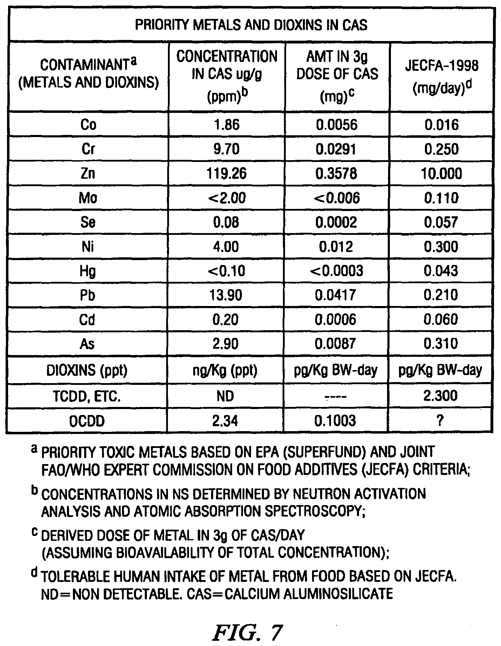

In animal studies with calcium montmorillonite clay, no adverse effects from clay treatment, at levels up to 2.0% (w/w) in the diet, have been reported. In recent studies in rodents, calcium montmorillonite clay were evaluated for potential toxicity and trace metal bioavailability in pregnant Sprague-Dawley rats throughout the period of gestation following high level exposure in the diet (2.0% w/w). Clays were supplemented in the balanced diet of Sprague-Dawley rats during pregnancy at a level of 2.0% (w/w). Evaluations of toxicity were performed on gestation day 16 and included maternal body weights, maternal feed intakes, litter weights, in addition to embryonic resorptions. Liver and, kidneys, tibia, brain, uterus, pooled placental, and pooled embryonic mass were collected and weighed. Tissue were lyophilized and neutron activation analysis (NAA) was then performed. Elements considered by NAA included: Al, Br, Ca, Ce, Co, Cr, Cs, Cu, Dy, Eu, Fe, Hf, K, La, Lu, Mg, Mn, Na, Nd, Ni, Rb, S, Sb, Sc, Se, Sm, Sr, Ta, Th, Te, Th, Ti, Tl, U, V, Yb, Zn, and Zr. Inductively coupled plasma-mass spectroscopy further confined that A1 was below detection limits (0.5ppm) in the brain, indicating no significant bioavailability of this metal from clay interactions in the GI tract. Animals supplemented with either clay were similar to controls with respect to toxicity evaluations and metal analysis, with the exception of decreased brain Rb following clay supplementation. Overall, the results of this study suggest that neither clay at high dietary concentrations, result in overt toxicity or influence mineral uptake or utilization in the pregnant rat. In some embodiments, clay was selected for testing due to its GRAS status and its purity priority trace metals and dioxin levels, see Figure 7. For example, the amount of heavy metal contamination in a derived dose of HSCAS clay is less than the Joint FAO/WHO Expert Commission on Food Additives (JECFA) criteria. More specifically, a derived dose 3g of CAS/day for Co, Cr, Zn, Mo, Se, Ni, Hg, Pb, Cd, As, and dioxins (TCDD and OCDD) is below the JECFA criteria.

Other studies in rodents and humans have confirmed the safety of calcium montmorillonite clay for application in human diets. In the rodent study, rats were fed rations containing about 0, 0.25, 0.5, 1.0, and 2.0% levels of calcium montmorillonite clay. Body weights, body weight gain, organ weights, histopathology, plasma biochemistry, serum vitamins A and E and micronutrients (Fe and Zn) were measured, standardized and compared to determine toxicity and any interactions of clay with critical nutrients at the end of the study. After 6 months exposure to clay, no morbidity or mortality was observed among treatment groups. There were no changes in the major organs, serum biochemistry or micronutrient levels. The ratios of organ weight to final body weight for the liver, kidneys, lungs, heart, brain, spleen, and tibia among the treatment groups in each sex were not significantly different histopathological analysis of the liver and kidneys indicated no differences between controls and clay treatments. These results suggest that inclusion of clay at levels less than 2.0 % (w/w) in the diet should not result in overt toxicity and can be used safely to reduce exposure aflatoxins in the gastrointestinal tract. In the human study, Calcium montmorillonite clay was initially tested for trace metals and dioxin content in order to confirm the composition of matter and ensure low levels of contamination.

Calcium montmorillonite clay was heat sterilized and packed into capsules for use in this particular example. The study design was based on 2 treatment groups: 1) low dose- 3 x 500 mg capsules x 3 times/day for a total of 2 weeks, and 2) high dose- 3 x 1,000 mg capsules x 3 times/day for a total of 2 weeks. The 2-week trial consisted of 50 healthy adults, age 22-40 selected by initial physical exams, laboratory analysis of biological fluids and questionnaire. One of ordinary skill in the art would be able to make capsules that are modified from the above description, that varied in dose, see Remmington's Pharmaceutical Sciences 17th Edition. Participants were then given clay capsules before meals with a bottle of spring water. Medical personnel were onsite to monitor any complaints or adverse effects. Blood and urine samples were taken at the end of the 2 week period and laboratory analysis and physical examinations were administered again. Any adverse events were reported according to NIH guidelines. Compliance with the dosing protocol reached 100% over the two-week study period. Analysis of clinical and biochemical data for side effects monitoring, blood, and urine parameters for liver and kidney function did not show any specific adverse effects.

Ingredient Description and Profile. Calcium aluminosilicate clay (CAS) has a different composition from hydrated sodium calcium aluminosilicate (HSCAS) clay, which has a dark tan color. The CAS has the appearance of an offwhite to gray-greenish colored free flowing powder. The CAS is odorless having a specific gravity of about 2.4. The isolated CAS is negligibly soluble in water and has a pH in the range of about 5-9 in suspension. Due to the silica and aluminum silicate components, the isolated CAS may have some adverse effects if dry particles are inhaled, but no adverse health effects are suspected from ingestion. The typical values are as follows:

**Typical Physical Properties:**

| | |
|---|---|
| Free Moisture (LOD) | 9% |
| Loose Bulk Density | 0.64 g/cc 40 lbs/ft3 |
| Packed Bulk Density | 0.80 g/cc 50 lbs/ft3 |
| Particle Size Distribution: | 5% +100 mesh |
| | 18% +200 mesh |
| | 60% +325 mesh |

**Typical Chemical Analysis:**

| | |
|---|---|
| Chemical Analysis by | %CaO 3.2 - 4.8 |
| X-Ray Fractionation (XRF) | %MgO 4.0 - 5.4 |
| Spectroscopy (weight %): | %Fe₂0₃ 5.4 - 6.5 |
| | %K₂0 0.50 - 0.90 |
| | %Na₂0 0.10 - 0.30 |
| | %MnO 0.01 - 0.03 |
| | %Al₂0₃ 14.8 - 18.2 |
| | %SiO₂ 62.4 - 73.5 |

Additionally, testing of the processed clay products from Engelhard's (now BASF), Jackson, MS plant have confirmed low levels of 2,3,7,8-tetrachlorodibenzo-p-dioxin (TCDD) in CAS (< 0.33 parts per trillion, ppt). TCDD is given in Engelhard (BASF) specifications as an index of the presence of dioxins in food ingredients.

Methods for COLE Index. A measure of expansive properties, the *coefficient of linear extensibility* (COLE) index is the ratio of the volume of a soil after wetting to the volume of soil before wetting minus one. COLE = (volume of clay after wetting/volume of clay before wetting) - 1 COLE index values greater than 0.03 indicate that significant smectite (swelling clay) is present in the sample. The general procedure can be summarized as follows:
1. Add 5 mL (5 cm³) of dry clay to a 25 mL graduated cylinder.
2. Add distilled water to the clay bringing the total volume to 25 mL.
3. Shake or stir suspension vigorously to ensure thorough wetting of clay.
4. Allow suspension to stand for 24 hr. at room temperature.
5. Measure the expanded volume of settled clay.

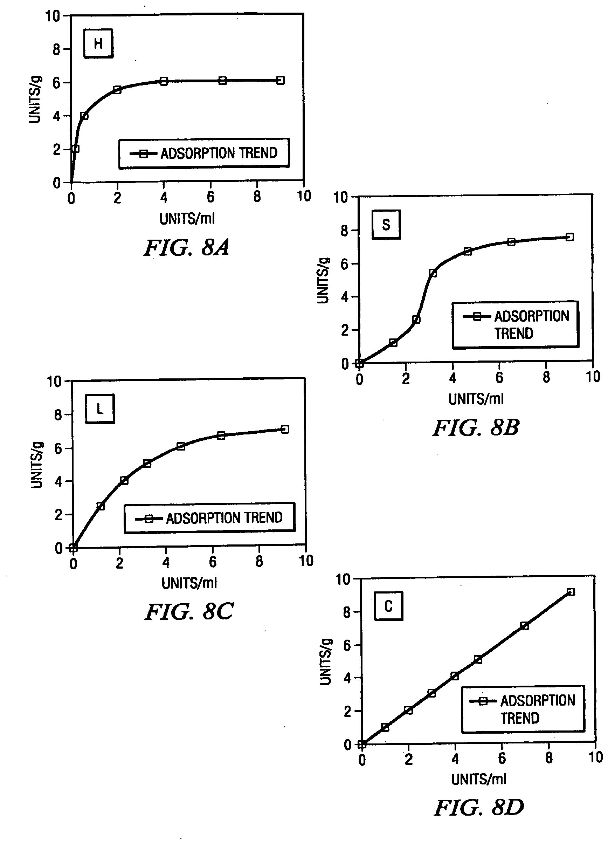

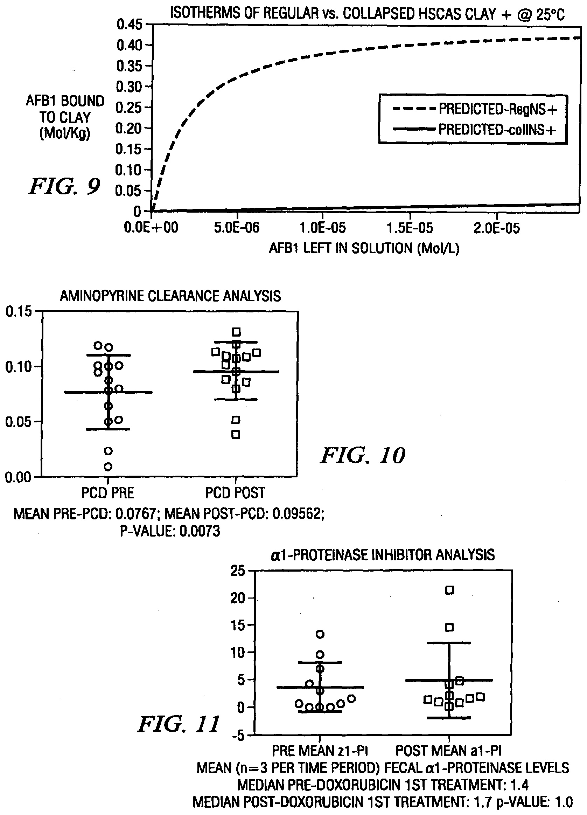

Shrink-swell potential correlates closely with the kind and amount of clay. The greatest shrink-swell potential occurs in soils that have high amounts of 2:1 lattice clays, such as smectites. Illitic clays are intermediate, and kaolinitic clays are least affected by volume change as the content in moisture changes. The isothermal analysis are shown in Figure 8. Adsorption curve characteristics are shown in graphs H or L, wherein, *r*² ≥ 0.90; *Qₘₐₓ* ≥ 0.25 mol/kg; *K*_{d} ≥ 1 x 10⁵; Δ*H_{ads}* minimum of - 20 kJ/mol and COLE index values: ≤ 0.8 after 24 hrs. Additionally, the isotherms of Regular vs. Collapsed HSCAS + at 25°C are shown in Figure 9.

### EXAMPLE 2

Primary hepatocellular carcinoma (HCC) results in between 250,000 and one million deaths globally per annum. HCC has unique geographic, sex, and age distributions which are likely determined by specific etiologic factors (i.e. hepatitis and environmental toxin exposure). The incidence of HCC varies widely according to geographic location . The distribution of HCC also differs among ethnic groups within the same country, and between regions within the same country.

High incidence regions (more than 15 cases per 100,000 population per year) include sub-Saharan Africa, the People's Republic of China, Hong Kong, and Taiwan. Over 40 percent of all cases of HCC occur in the People's Republic of China, which has an annual incidence of 137,000 cases. In contrast, North and South America, most of Europe, Australia and parts of the Middle East are low incidence areas with fewer than three cases reported per 100,000 population per year. However, the incidence in the United States has increased during the past two decades, possibly due to a large pool of people with longstanding chronic hepatitis.

Males are far more likely to develop HCC than females, and the disparity is more pronounced in high incidence regions, where males are affected 2.1 to 5.7 times more frequently than females (mean 3.7:1). The ratio decreases to a mean of 2.4:1 in intermediate incidence areas, and is lower in low incidence regions. Although not fully understood, these differences in sex distribution are thought to be due to variations in hepatitis carrier states, exposure to environmental toxins, and the trophic effect of androgens.

The majority of HCCs occur in patients with chronic liver disease or cirrhosis. Thus, older patients with longstanding liver disease are more likely to develop HCC. Several large prospective studies conducted in both Asia and western Europe have noted a mean age at presentation between 50 and 60 years. In sub-Saharan Africa, however, the mean age of presentation of HCC is decreasing, with a mean age of 33 years at presentation.

Efforts to understand the unique distribution of HCC have augmented our understanding of the risk factors for the development of this disease. Thus, a variety of important risk factors for the development of HCC have been identified. These include the hepatitis B carrier state, environmental toxins, chronic hepatitis C virus (HCV) infection, hereditary hemochromatosis, and cirrhosis of almost any cause. However, HCC can also occur in patients without known risk factors. The role for surveillance in any of these disorders is discussed separately.

Hepatitis B Carrier State. The association between the hepatitis B carrier state and hepatocellular carcinoma has been demonstrated in several large population based studies and in other reports. In one report, for example, 22,707 male government employees in Taiwan, 15 percent of whom were HBV carriers (hepatitis B surface antigen positive), were followed between 1975 and 1978. The relative risk of HCC in these HBsAg carriers was 223 times that of noncarriers. In another series, the relative risk of HBsAg was 6.9 among 917 Japanese patients with cirrhosis or chronic hepatitis.

Environmental Toxins. At least two environmental toxins, aflatoxin and contaminated drinking water, may contribute to the pathogenesis of HCC. *Aflatoxin* is a mycotoxin that commonly contaminates corn, soybeans, and peanuts. High rates of dietary aflatoxin intake have been associated with HCC. As an example, the Penghu Islets in Taiwan have an extremely high incidence of HCC which is not entirely accounted for by the HBV carrier state. In a study in which 20 patients with HCC from this region were compared to 86 age-matched controls, the patients were more likely to have aflatoxin B1-albumin adducts (65 versus 37 percent; adjusted odds ratio 5.5); 94 percent of the patients were HBsAg carriers. In another study from Shanghai, the odds of developing HCC among individuals with HBV and exposure to aflatoxin was 59.4 times the normal population incidence.

Mutations of the p53 tumor suppressor gene have been demonstrated in patients with hepatocellular carcinoma who have chronically been exposed to aflatoxin. Similar findings also have been demonstrated in animal models of hepatocarcinogenesis in which p53 mutations have been observed in laboratory animals exposed to HBV and aflatoxins. The potentiating effect of these risk factors has also been demonstrated in transgenic mice that express hepatitis B surface antigen; in one study, some of these mice were bred to lack one of the p53 alleles and/or were exposed to aflatoxin. At 13 months of age, high-grade HCC developed in all seven mice with each of the three risk factors compared to 62 percent of mice with both p53 alleles even though they were exposed to aflatoxin and 25 percent of mice lacking one p53 allele but not exposed to aflatoxin.

Additionally, *contaminated drinking* water has been linked to HCC. For example, several studies conducted in rural China have noted a higher mortality rate from HCC among people who drink pond-ditch water compared to those who drink well water (100 versus fewer than 20 deaths per 100,000 population per year). The blue-green algal toxin Microcystin commonly contaminates these ponds and is thought to be a strong promoter of HCC.

Knowingly eating and drinking environmental toxins that are established causative agents for HCC is risky. However, many citizens of the world having low socioeconomic means usually have a choice between ingesting the contaminated food, or not eating at all. Given this choice, the risk of possible HCC outweighs starvation and certain death.

The effects of environmental toxins in persons predisposed to HCC can be mitigated by administering orally an effective amount of an isolated calcium aluminosilicate clay in a powder form capsule at least once per day, preferably before, during, or after each meal. The isolated calcium aluminosilicate clay is substantially free from T4-dioxin and toxic heavy metal contamination, and is capable of binding the environmental toxins (e.g. aflatoxin or microcystin). In a preferred embodiment, the isolated calcium aluminosilicate clay has a chemical composition comprising: CaO above 3.2%; MgO about 4.0 - 5.4%; Fe₂0₃ about 5.4 - 6.5; K₂0 about 0.50 - 0.90%; Na₂0 about 0.10 - 0.30%; MnO about 0.01 - 0.03%; Al₂0₃ about 14.8 - 18.2%; and SiO₂ about 62.4 - 73.5%; wherein the chemical composition is determined by x-ray fractionation as a weight percent. Additionally, the isolated calcium aluminosilicate clay has an average particle size that is less than about 100 microns, preferably about 80 microns.

### EXAMPLE 3

In medicine, tumor necrosis factor alpha (TNFα, cachexin or cachectin) is an important cytokine involved in systemic inflammation and the acute phase response. TNFα was isolated in 1975 by Carswell *et al* as a soluble factor released by host cells that caused necrosis of a transplanted tumor, "sarcoma Meth A". Although TNFα does cause the necrosis of some tumors, it may stimulate the growth of others. In that sense, the name is somewhat of a misnomer.

TNFα is a member of a group of other cytokines that all stimulate the acute phase reaction. It is a 185 amino acid glycoprotein peptide hormone, cleaved from a 212 amino acid-long propeptide on the surface of macrophages. Some cells secrete shorter or longer isoforms. Genetically it maps to chromosome 6p21.3 in humans.

TNFα is released by white blood cells, endothelium and several other tissues in the course of damage, e.g. by infection. Its release is stimulated by several other mediators, such as interleukin 1 and bacterial endotoxin. TNFα has a number of actions on various organ systems, for example: stimulating of the hypothalamic-pituitary-adrenal axis by stimulating the release of corticotropin releasing hormone (CRH); Suppressing appetite (hence its name "cachexin" - cachexia is severe weight loss in illness); On the liver: stimulating the acute phase response, leading to an increase in C-reactive protein and a number of other mediators; It attracts neutrophils very potently, and helps them to stick to the endothelial cells for migration; On macrophages: TNFα stimulates phagocytosis, and production of IL-1, oxidants and the inflammatory lipid, prostaglandin E2 (PGE2). A locally increasing concentration of TNFα will cause the cardinal signs of Inflammation to occur: Heat, swelling, redness and pain.

The inhibition of TNFα with a monoclonal antibody or a circulating receptor such as infliximab (Remicade®), etanercept (Enbrel®), or adalimumab (Humira®) is used in modem treatment of various autoimmune disorders such as rheumatoid arthritis, Crohn's disease and psoriasis. Clinical trials regarding the effectiveness of these drugs on hidradenitis suppurativa are currently ongoing.

Such drugs may raise the risk of contracting tuberculosis or causing a latent infection to become active. Infliximab and adalimumab (HUMIRA®) have label warnings which state that patients should be evaluated for latent TB infection and treatment should be initiated prior to starting therapy with these medications. TNF-α or the effects of TNF-α are also inhibited by a number of natural compounds, including curcumin (an ingredient in turmeric) and catechins (in green tea).

Additionally, TNF-α is produced primarily by monocytes and macrophages. It is found in synovial cells and macrophages in the tissues. It shares many properties with another cytokine - interleukin 1. TNF-α occurs in many inflammatory diseases, and also as a response to endotoxins from bacteria for example. Generally, TNF-α causes stimulation of IL1 and GM-CSF; increased tissue damage by IL1; induction of collagenases by fibroblasts and chondrocytes; plays a role in modulating HLA class 2 expression, as well as the adhesion molecules.

The receptors for the TNF-α are on several mononuclear cells, in the synovial membrane, as well as the peripheral blood and synovial fluid. There are also soluble receptors - receptors that are free in solution. The soluble TNF-α receptors can block the TNF-α by mopping up and blocking the levels of TNF-α, so that less is available to activate the mononuclear cells. They thus act as natural inhibitors. The incidence and extent of the levels of the soluble receptor correlates with disease activity.

The therapeutic use of the soluble receptor is restricted because the half life of the molecule is short, and thus combinations of the soluble receptor with immunoglobulin or other molecules has been attempted to try and increase the half life to enable therapeutic applications. It is the combination of recombinant soluble form of human p75 TNFR to the Fc fragment of IGG, that is known as etanercept or ENBREL®, that is made by Immunex Corporation (Thousand Oaks, CA).

TNF-α also plays a central role in the pathogenesis of mucosal inflammation in Crohn's disease. Therapy with the chimeric monoclonal antibody to TNF (infliximab) has profoundly changed the management of refractory luminal and fistulizing Crohn's disease. Over two-thirds of patients with Crohn's disease treated with infliximab (REMICADE®) achieve remission and maintenance therapy has been approved on account of its corticosteroid-sparing efficacy in refractory luminal and fistulizing Crohn's disease.

The efficacy in Crohn's disease provided the rationale for clinical trials of infliximab (and other anti-TNF agents) in patients with ulcerative colitis, a disorder in which TNF may also have an important role. TNF-α is expressed at high levels in the colonic mucosa of patients with ulcerative colitis (UC). There is also an increased production of TNF-α by colonic lamina propria mononuclear cells and high concentrations of TNF-α in stools, rectal dialysates, and urine of patients with UC.

A potential role for anti-TNF therapy in UC was first supported by studies on cotton-top tamarins, an animal that develops spontaneous diffuse non-granulomatous colitis in captivity. Treatment with a humanized monoclonal antibody to TNF-α, CDP571, resulted in significant clinical and histological improvement. However, monoclonal antibodies are expensive to produce for chronic therapies and other anit- TNF-α agents are continually being sought. For example, in a pilot study, 15 patients with mild-to-moderate active ulcerative colitis were treated openly with a single intravenous infusion of CDP571 (Humicade, a humanized IgG4 antibody) and monitored for eight weeks. Treatment was well tolerated and plasma half-life of CDP571 was approximately seven days. There was a significant reduction in clinical activity by one week post-infusion, but only a modest (and non statistically significant) reduction was observed at two weeks. Mixed results have also been seen with this drug in treatment of Crohn's disease.

The clay of this invention effectively binds TNF-α. In this invention, absorption of TNFα by CAS was determined using an ELISA assay. The materials used were as follows: Recombinant TNF-α (50 mg/ml stock in 100% ddH₂O); TNF-α ELISA kit (R&D Systems Inc.); CAS; and Phosphate buffered saline (PBS). The samples were prepared by allowing ELISA kit components to warm to room temperature before beginning experiment.

The CAS samples were prepared by suspending 6 different concentrations of CAS in PBS. CAS concentrations chosen for this experiment are 0.5 mg/ml, 1 mg/ml, 5 mg/ml, 10 mg/ml, 50 mg/ml, and 100 mg/ml. Recombinant TNF-α was added to each of the above samples at a final concentration of 1000 pg/ml TNF-α. At the same time, one sample of 1000 pg/ml TNF-α in 100% PBS (no CAS) was prepared. The samples were mixed using a vortex mixer for 30 seconds and allowed to incubate at room temperature for 30 minutes. During this incubation samples were mixed every 10 minutes for 5 seconds with a vortex mixer. Samples were then centrifuged at 10,000 rpm for 5 minutes and the supernatant was isolated. The standard protocol for ELISA was followed using these supernatants and samples.

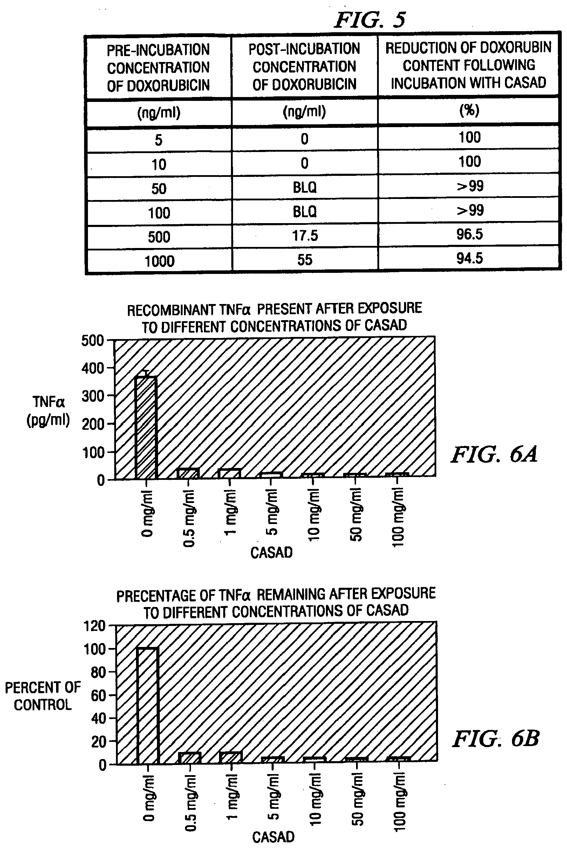

As shown in Figure 6A, there was a significant reduction in the concentration of TNF-α in the solution after exposure to all of the concentrations of CAS. Figure 6B shows the reduction of TNF-α as a percentage of the control sample that contained no CAS.

### EXAMPLE 4

Diarrhea is a common complication of both cancer and its treatment. In the canine patient, diarrhea may be the result of dietary indiscretion, disease related, or treatment related (due to chemotherapy or radiation therapy). While most cases of diarrhea are self limiting or respond to minimal treatment, in the cancer patient, additional therapy may be warranted. Many animals undergoing therapy will have repeated exposure to cytotoxic chemotherapy or radiation therapy. Continued treatment with anti-secretory agents such as loperamide or antibiotics such as metronidazole is not without the potential risk of serious complications. Loperamide can result in dry mouth and nausea and in the long term setting can lead to dependence. Metronidazole, when given long term, carries an increased risk of neurological complications. Agents such as CASAD, which are not absorbed through the gastrointestinal tract, may provide a safer option for chronic administration.

Diarrhea in the canine patient not only affects the patient, but also the family unit. Diarrhea results in increased stress on the caretaker, by requiring additional work such as additional trips outside for bowel movements, cleanup of fecal accidents, and administration of medication, rehydration solutions and food preparation. Smectite clays in the pediatric model have been shown to decrease the duration of watery stools; therefore, decreasing the work load and strain on the family unit. Decreasing the strain on the family unit, thus promotes greater compliance and willingness to treat. Quality of life for the patient is also improved by decreasing the duration of a post therapy diarrhea episode.

Dosages of chemotherapy are limited by toxicity. Patients are given chemotherapy based on the maximally tolerated dose. A common dose limiting toxicity is diarrhea (both large and small bowel). A chemotherapy which has a high incidence of chemotherapy induced colitis is doxorubicin. By reducing the incidence and duration of diarrhea, the maximally tolerated dose may be increased allowing for improved response and survival.

The most commonly recommended therapeutic for chemotherapy or radiation therapy induced diarrhea is metronidazole. Metronidazole is a synthetic nitroimidazole with antibacterial, anti-protozoal and anti-inflammatory properties. It should be used cautiously in humans and animals with impaired liver function as it is metabolized by the liver. Neurotoxicity has been associated with higher doses and chronic administration.

Alternatively sulfasalazine has been recommended for the treatment of chemotherapy induced diarrhea. Sulfasalazine is an antibiotic with antibacterial and anti-inflammatory properties. Potential adverse reactions include keratoconjuncitivitis sicca (KCS), hepatotoxicity, hemolytic anemia and leukopenia. Animals, specifically black and tan dog breeds, are at an increased risk of adverse reactions.

Dioctahedral magnesium smectite is a naturally occurring clay composed of fine sheets of aluminomagnesium silicate. This composition has not only been used in many other countries for the treatment of pediatric acute infectious diarrheas; but has also been reportedly used for the management of chronic diarrhea conditions such as inflammatory bowel disease, Cohn's disease and food allergies. The commonly reported mechanism of action of smectite clay is the adsorption of luminal toxins, antigens and bacteria. Other proposed mechanisms of action include modifications of the rheological properties of the gastrointestinal mucosa, anti-inflammatory properties, increased secretion of mucopolysaccharide 2 (MUC2), and restoration of luminal integrity.

Recent work by Gonzalez *et al*., shows that in the rat model, smectite clay down-regulated the inflammatory response; and reduced the levels of myeloperoxidases and IL-1β, which indicates decreased infiltration and activity of neutrophils and monocytes in the intestinal tissue samples. Smectite clay also increased the secretion of MUC2 in the colon thus providing an improved barrier to luminal contents. Additionally, smectite clay inhibited the basolateral secretion IL-8 in a dose dependant manner. The results suggest that smectite clay was as effective as sulfasalazine in an experimental model of chronic colitis.

In the rabbit model for experimental infectious diarrhea, smectite clay was shown to decrease bacterial mucolysis and the destruction of the luminal surface membranes by pathogenic bacteria. *Escherichia coli* 0128B12 was used to create an invasive and toxigenic situation to attempt to determine the mechanism of action of smectite clay. Smectite clay was shown to favor toxin absorption while allowing absorption of luminal electrolytes. In addition, membrane enzymes levels of disaccharidase and alkaline phosphatase were both elevated in the smectite clay model which is consistent with protective effects on the luminal surface in the presence of an invasive, toxigenic bacteria.

In the pediatric model, smectite clay has been used to treat acute infectious diarrhea. In an open, randomized, multi-center trial in Lithuania, the duration of diarrhea was significantly shorter when smectite clay was used in combination with an oral rehydration solution then when treated with an oral rehydration solution alone (42.3+/- 24.7 hours versus 61.8 +/-33.9 hours). A similar double blinded placebo controlled study in Egypt found that although smectite clay did not impact the initial volume of stool; it decreased the duration of diarrhea from 73 hours to 53 hours when used in combination with oral rehydration.

Most sources of dioctahedral smectite contain contaminants, such as toxic heavy metals and dioxin that prohibit long term or high dose use. Most of these smecite clays contain sodium or magnesium as the primary interlayer cation in the crystal structure. These ions can have a negative effect on critically ill patient's electrolyte balance. Additionally, most of the clay mentioned above were not well characterized or were of varying particle size and moisture content. In the critically ill patient heavy particles tend to settle out and are retained in the gastrointestinal tract resulting in material retention due to poor gut motility.

The isolated clay used in this invention is a unique sterile calcium aluminosilicate with a mean particle size of approximately 100 microns or less, low sodium content, and calcium aluminosilicate anti-diarrheal clay, coded CASAD. This isolated clay has a high Ca/Mg content and was substantially free of T4 dioxin, toxic heavy metals and contained no binders or fillers. The isolated clay also a low sodium content when compared to other calcium-based clays and has none of the disadvantages associated with clays that have been described in detail above. The isolated clay was formed into 500 milligram (mg) tablets that were individually packaged in laminated foil. Pills having a diameter of about 1 cm were formed using a tablet press machine (Stokes Pennwalt Tablet Press, Warminster, PA).

Historically, death has been reported in connection with the acute and short-term toxicosis associated with the administration of doxorubicin to dogs with malignant tumors For example, in one example, 185 dogs with histologically confirmed, measurable malignant tumors were used in a study to determine the toxicity of the anthracycline antitumor antibiotic, doxorubicin, which was administered once or twice (at a 21-day interval) at the rate of 30 mg/m² of body surface area, iv. During this study, 7 dogs died as a direct result of doxorubicin-induced toxicosis and 16 died as a direct result of the malignant neoplastic disease. Each dog was evaluated for signs of toxicosis for 3 weeks after the last dose was administered (15 dogs received 1 dose, 170 dogs received 2 doses) or until the dog died, whichever came first. The most common signs of toxicosis were vomiting, diarrhea, colitis, anorexia, and pruritus. The probability of doxorubicin-induced toxicosis decreased significantly (P less than 0.0001) in inverse relationship to body weight. Dogs with signs of toxicosis during the 21-day interval from administration of the first dose of doxorubicin were 17.2 times (P less than 0.01; 95% confidence interval; 5.5, 54.2) more likely to develop signs of toxicosis during the 21-day interval from the second dose of doxorubicin. The performance status of each dog was evaluated using a modified Karnofsky performance scheme; the only time the performance status was adversely affected to a significant extent by doxorubicin-induced toxicosis was during the 21-day period, starting with the second dose (P less than 0.0001). (Ogilvie GK, et al., "Acute and Short-term Toxicoses Associated with the Administration of Doxorubicin to Dogs with Malignant Tumors." Am Vet Med Assoc. 1989 Dec 1;195(11):1584-7).

Intractable diarrhea in cancer bearing dogs is defined as persistent loose watery stools without resolution despite using standard treatment measures such as metronidazole, sulfasalazine or dietary approaches for a period of no less than 48 hours. In another example, a total of 19 cancer bearing dogs were treated with CASAD. Of the 19 dogs, 16 had sufficient data for analysis, with three animals having incomplete data. Six patients had diarrhea that was not considered to be treatment related. In this grouping, 5 of the 6 patients had resolution of their clinical signs, with one patient having an incomplete data set. Causes of diarrhea in this grouping include: dietary indiscretion, stress colitis, and as a result of the neoplastic process. These patients had an average of 4.2 days of diarrhea prior to starting CASAD. One of these patients had a 14 day history of diarrhea prior to starting CASAD, which resolved within 3 days of starting CASAD. Most patients received at least one (1) course of metronidazole prior to starting the trial. When appropriate, fecal flotation with ZnSO₄ and cultures were performed. Patients were then placed on the CASAD trial and received 500 mg orally every six (6) hours until there were two (2) consecutive formed stools. Resolution of diarrhea was defined as two consecutive formed stools. Data recorded include the date and type of the most recent chemotherapy, duration of diarrhea prior to starting CASAD and time to resolution of diarrhea. The average time to resolution of ID was 3.2 days in the patients with known response.

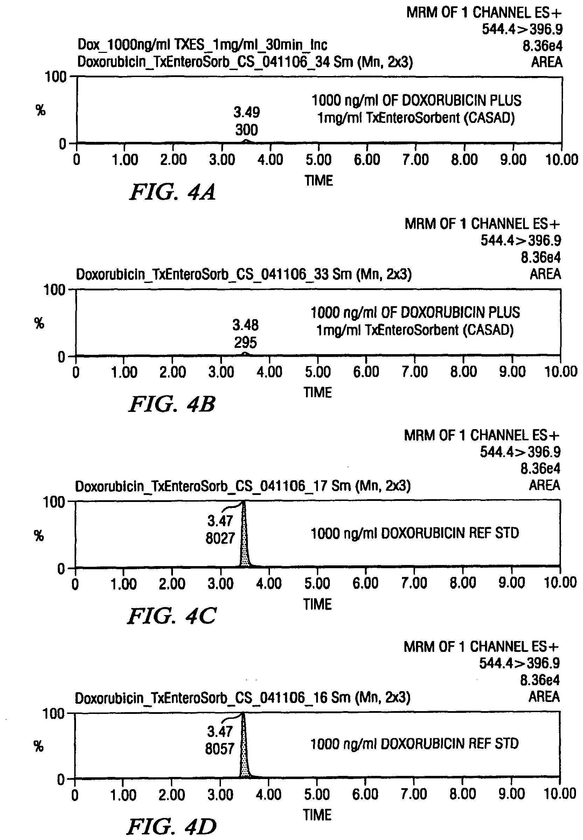

The current invention was also utilized, in a further example, on thirteen patients where chemotherapy was the inciting cause of diarrhea, with doxorubicin being the most commonly reported cause (n=5). Other various chemotherapy agents were also reported to include: vincristine (2), cyclophosphamide (2), lomustine (2), vinblastine (1), and carboplatin (1). Patients had a mean of 5.2 days between chemotherapy administration and onset of diarrhea which lis consistent with GI toxicity from cytotoxic chemotherapy. Patients had an average of 4.4 days of diarrhea prior to being started on CASAD. Most owners reported improvement within 48 hours and complete resolution of signs in 2.9 days. Absorption of doxrubicin by CASAD was determined using a mass spectrum assay, as shown in Figure 4. Figure 5 shows that 1mg/ml of CASAD absorbs greater than 99% of doxrubicin from solution when the drug concentration is 100ng/ml or less. Even at relatively high drug concentration of 1000 ng/ml (1 microgrma/ml) 95% of drug is removed from solution by CASAD.

CASAD was well tolerated with only one adverse reaction reported. One patient experienced constipation as a result of treatment with CASAD. No other adverse reactions were reported during the study period.

Overall, CASAD was found to be well tolerated with only one adverse event reported during the treatment period. This patient experienced constipation as a result of treatment. This condition quickly resolved with discontinuation of CASAD.

Based on the results of these examples, CASAD may be used as a preventive for radiation therapy and chemotherapy induced diarrhea. Smectite clay can delay the onset of radiation therapy induced colitis in the human patient. CASAD may be an effective preventative or even decrease the duration of treatment induced diarrhea, the maximally tolerated dose of radiation or chemotherapy can be increased and; therefore, potentially improve response rates and survival times.

### EXAMPLE 5

A 56 year old white female that had been diagnosed with irritable bowel syndrome for over 40 years and continued to experience episodic bouts of profuse watery diarrhea especially when ingesting certain complex starch containing foods. These diarrhea episodes were preceded by abdominal cramps and pain that continued for several hours.

One 500 mg tablet of CASAD was offered to the subject immediately after a diarrhea episode. She reported that the abdominal pain and cramping went away within 30 minutes of ingestion of the CASAD. She went to bed after the pain had subsided and reported 8 hours of very restful sleep. In the morning she reported that she had a formed stool. She did not experience a diarrhea episode for over two weeks which she reported as "unusual."

Subsequently, she had occasion to begin to experience abdominal cramping and pain which she reported to be an indication that a diarrhea episode was about to occur within 1 hour. She was offered another 500 mg tablet of CASAD that she readily ingested. She reported that the cramps and pain subsided in about 30 minutes and that no diarrhea episode occurred. Since this time, if she has a cramp and/or pain and ingests one 500 mg tablet of CASAD with at least 100 ml of water, she does not have a diarrhea episode. In a 60 day period she ingested five 500 mg tablets without adverse event and reported no episodes of diarrhea.

There are the advantages of using clay of a certain particle size that has low levels of heavy metals and dioxins, including (a) lower toxicity; (b) increased surface area for sorption; (c) increased surface area for coating; (d) increased number of particles to attach or bind with toxic particles or molecules in the environment. The tablets also dissolve rapidly in the digestive tract.

The therapeutic advantages of using a small particle size CAS that is low in toxic heavy metals and dioxin is that the toxicity of the composition when compared to clay is reduced. Examples have shown that some level of heavy metals and dioxins are present in raw clay, but by screening for particle size, it is possible to cut the toxin level significantly, thus decreasing toxicity and making the product safer. The smaller particle size has the added benefit of increasing the surface area and allowing for more adsorption of diarrhea-causing toxins in the gastro-intestinal tract ("GIT"). There is increased surface area for coating the GIT. Additionally there are more CAS particles to bind to toxic particles or molecules in the environment of the GIT.

The tablet dose form has the therapeutic advantage in that tablets can be swallowed and dissolve rapidly in the stomach. It is desirable to have dissolution of the tablet as fast as the stomach allows. The CASAD is to be thoroughly mixed with the GIT contents. This dose site and mixing action gives CASAD the maximum opportunity to adsorb toxic substances in the GIT. Moreover, the tablet can be administered with proper dosage.

### EXAMPLE 6

Dogs with hepatic disease have a decreased hepatic demethylating capacity. Doxorubicin is a chemotherapeutic agent that is commonly used for the therapy of dogs with malignant neoplasms. The goal of this example was to evaluate the effect of a single administered dosage of doxorubicin on hepatic demethylation capacity.

A ¹³C-aminopyrine demethylation blood test was performed in 16 dogs before and after undergoing chemotherapy with doxorubicin for malignant neoplasia. A baseline blood sample of 1 ml was taken, followed by intravenous injection of 2 mg/kg ¹³C-aminopyrine and collection of another 1 ml blood sample at 45 minutes after ¹³C-aminopyrine administration. Blood samples were immediately placed into vacutainer tubes containing 2 ml of 6M hydrochloric acid and the percent dose of the ¹³C administered as ¹³C-aminopyrine was determined based on fractional mass spectrometry. Two weeks after doxorubicin treatment the ¹³C-aminopyrine demethylation blood test was repeated in the same fashion and the results compared to those obtained prior to chemotherapy by a paired *t*-test. A p-value <0.05 was considered statistically significant.

The results are shown in Figure 10. The reference range established in 45 healthy dogs was 0.08 to 0.200. None of the 16 dogs showed any clinically obvious side affects from the ¹³C-aminopyrine demethylation blood test before or after doxorubicin administration. The clearance at 45 minutes after ¹³C-aminopyrine administration increased in all dogs after treatment with doxorubicin. The mean±SD clearance at 45 minutes after ¹³C-aminopyrine administration was 0.0767±0.0253 before the first doxorubicin administration and was significantly increased 14 days later (prior to the second doxorubicin administration) to 0.09562±0.0074 (p-value = 0.0073).

The results indicate that doxorubicin treatment in dogs leads to a significant decrease in hepatic demethylation capacity. It is unclear if this damage occurs as a direct effect on the hepatocyte during drug administration or occurs as an indirect effect of hepatocyte exposure to active and inactive doxorubicin metabolites via enterohepatic recirculation. The administration of CASAD will reduce the enterohepatic cycling of doxorubicin and metabolites, thus reducing hepatoxicity and patient morbidity.

### EXAMPLE 7

Fecal alpha(1)-proteinase inhibitor (α-1PI) clearance is a reliable, noninvasive marker for protein-losing enteropathy in animals and canines. Furthermore, the canine gut is a good model for the human gut, so results of testing on dogs can be considered relevant to humans. In this example, a number of dogs were tested. Fecal samples were collected daily for 3 days following each Adriamycin administration. An ELISA was used to measure α-1PI concentrations in fecal extracts.

Fecal samples were available for analysis following both chemotherapy treatments from 13 dogs. The results are shown in Table 1 below. Fecal α-1PI concentrations, expressed as micro g/g of feces, were not significantly different following the two treatments. However, fecal α-1PI concentrations (mean, median, minimum-maximum) were significantly higher in dogs with gastrointestinal symptoms (diarrhea, vomiting, appetite loss) than in dogs without these symptoms (shadow boxes indicate symptomatic episodes).

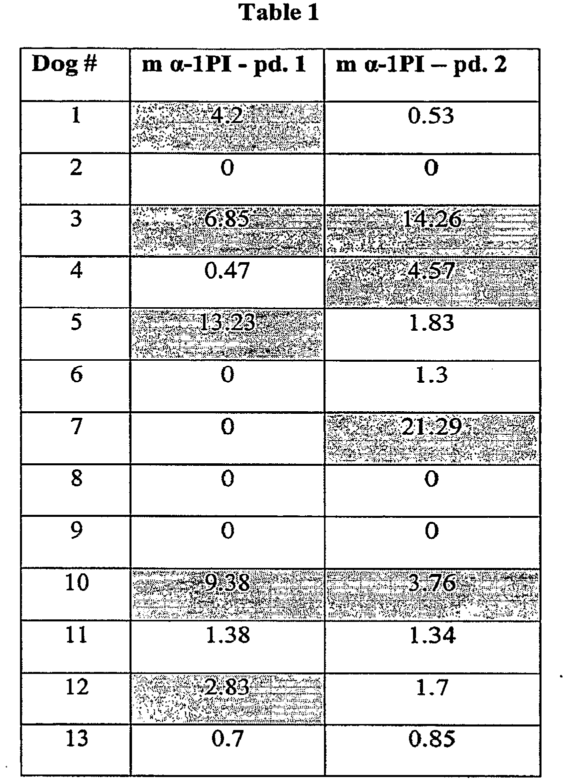

The use of CASAD will abrogate inflammation, reduce the concentration of fecal α-1PI concentration in the feces, and thereby reduce patient morbidity. The ELISA results will confirm CASAD's effectiveness by showing a decrease in the α-1PI biomarker.

### EXAMPLE 8

The clinical course of inflammatory bowel disease (IBD) in dogs is characterized by spontaneous exacerbations and remissions, which makes assessment of disease burden difficult. A previous study in 58 dogs provided validation for an objective scoring system to assess IBD in dogs and the response to treatment.

Among 14 dogs with sufficient medical record and owner communication information, the canine inflammatory bowel disease activity index (CIBDAI) showed good correlation with patient morbidity. The CIBDAI is a reliable measure of inflammatory activity in canine intestinal inflammation and the CIBDAI can be used to measure the ability of CASAD to treat or to prevent canine inflammatory bowel disease. Table 2 below shows the results of the study. The criteria used for the scoring in the table is found at the bottom of the table. Dogs in the study were given a "palliative" Adriamycin dosage (30 mg/m2, IV every 14 days). Symptoms related to intestinal inflammation are expected in fewer than 10% of treated dogs.

**Table 2**

| Dog # | | Pre Adria 1 | Post Adria 1 | Pre Adria 2 | Post Adria 2 |
|---|---|---|---|---|---|
| | | | | | |
| Dog 1 | | A.0 | A.0 | A.0 | **A.2** |
| | | B.0 | B.0 | B.0 | B.0 |
| | | C.0 | C.0 | C.0 | C.0 |
| | | D.1 | D.0 | D.0 | D.0 |
| | | E.0 | E.0 | E.0 | E.0 |
| | | F.0 | F.0 | F.0 | F.0 |
| | Day 21 - Owner reported dumpty for 3 days post Adria #2 | | | | |
| | | | | | |
| Dog 2 | | A.0 | A.0 | A.0 | A.0 |
| | | B.0 | B.0 | B.0 | B.0 |
| | | C.0 | C.1 | C.0 | C.0 |
| | | D.0 | D.0 | D.0 | **D.2** |
| | | E.0 | E.0 | E.0 | **E.2** |
| | | F.0 | F.0 | F.0 | F.0 |
| | Day 21 - Diarrhea started, treated with Flagyl, resolved in 2 days | | | | |
| | | | | | |
| Dog 3 | | A.0 | A.0 | A.0 | A.0 |
| | | B.0 | B.0 | B.0 | **B.2** |
| | | C.0 | C.0 | C.0 | C.0 |
| | | D.0 | D.0 | D.0 | D.0 |
| | | E.0 | E.0 | E.0 | E.0 |
| | | F.0 | F.0 | F.0 | F.0 |
| | | | | | |
| Dog 4 | | A.0 | A.0 | A.0 | A.0 |
| | | B.0 | B.0 | B.0 | B.0 |
| | | C.0 | C.0 | C.0 | C.0 |
| | | D.0 | D.0 | D.0 | D.0 |
| | | E.0 | E.0 | E.0 | E.0 |
| | | F.0 | **F.2** | F.0 | F.0 |
| | | | | | |
| Dog 5 | | **A.2** | NA | NA | NA |
| | | B.0 | NA | NA | NA |
| | | C.0 | NA | NA | NA |
| | | D.0 | NA | NA | NA |
| | | E.0 | NA | NA | NA |
| | | F.0 | NA | NA | NA |
| | Patient was euthanized 5 days after initial treatment due to chemotherapy-induced complications. | | | | |
| | | | | | |
| Dog 6 | | A.0 | A.0 | A.0 | **A.3** |
| | | B.1 | B.0 | B.0 | **B.3** |
| | | C.0 | C.0 | C.0 | C.0 |
| | | D.0 | D.0 | D.0 | D.0 |
| | | E.0 | E.0 | E.0 | E.0 |
| | | F.0 | F.0 | F.0 | F.0 |
| | Patient was euthanized on Day 15 for hemoabdomen | | | | |
| | | | | | |
| Dog 7 | | A.0 | A.0 | A.0 | A.0 |
| | | B.0 | B.0 | B.0 | B.0 |
| | | C.0 | C.0 | C.0 | C.0 |
| | | D.0 | D.0 | D.0 | D.0 |
| | | E.0 | E.0 | E.0 | E.0 |
| | | F.0 | F.0 | F.0 | F.0 |
| | | | | | |
| Dog 8 | | A.0 | **A.1** | A.0 | A.0 |
| | | B.0 | **B.2** | **B.1** | **B.2** |
| | | C.0 | **C.2** | C.0 | C.1 |
| | | D.0 | **D.3** | D.0 | D.3 |
| | | E.0 | **E.2** | E.0 | E.2 |
| | | F.0 | F.0 | F.0 | F.0 |
| | Day 7 - Diarrhea, not eating, vomited once | | | Day 21 - Diarrhea for 3 days, no eating for 3 days | |
| | | | | | |
| Dog 9 | | A.0 | A.0 | A.0 | A.0 |
| | | B.0 | B.0 | B.0 | B.0 |
| | | C.0 | C.0 | C.0 | C.0 |
| | | D.0 | **D.1** | **D.1** | D.0 |
| | | E.0 | E.0 | E.0 | E.0 |
| | | F.0 | F.0 | F.0 | F.0 |
| | Patient had consistent soft stools - even in the weeks prior to treatment. | | | | |
| | | | | | |
| Dog 10 | | A.0 | A.0 | A.0 | A.0 |
| | | B.0 | B.0 | B.0 | B.0 |
| | | C.0 | C.0 | C.0 | C.0 |
| | | D.0 | D.0 | D.0 | D.0 |
| | | E.0 | E.0 | E.0 | E.0 |
| | | F.0 | F.0 | F.0 | F.0 |
| | | | | | |
| Dog 11 | | A.0 | **A.1** | A.0 | A.0 |
| | | **B.1** | **B.2** | B.0 | B.0 |
| | | C.0 | **C.2** | C.0 | C.0 |
| | | D.0 | **D.2** | D.0 | D.0 |
| | | E.0 | **E.1** | E.0 | E.0 |
| | | F.0 | F.0 | F.0 | F.0 |
| | Adria #2 delayed due to vomiting, diarrhea, decreased appetite and thrombocytopenia | | | | |
| | | | | | |
| Dog 12 | | A.0 | A.0 | A.0 | A.0 |
| | | B.0 | B.0 | B.0 | B.0 |
| | | C.0 | **C.1** | C.0 | C.1 |
| | | D.0 | D.0 | D.0 | D.0 |
| | | E.0 | E.0 | E.0 | E.0 |
| | | F.0 | F.0 | F.0 | F.1 |
| | | | | | |
| Dog 13 | | A.1 | A.0 | A.0 | A.0 |
| | | B.0 | B.0 | B.0 | B.0 |
| | | C.0 | C.0 | C.0 | C.0 |
| | | D.0 | D.0 | D.0 | D.0 |
| | | E.0 | E.0 | E.0 | E.0 |
| | | F.0 | F.0 | F.0 | **F.1** |
| | | | | | |
| Dog 14 | | A.0 | A.0 | A.0 | **A.1** |
| | | B.0 | B.0 | B.0 | **B.1** |
| | | C.0 | C.0 | C.0 | C.0 |
| | | **D.1** | D.0 | D.0 | D.0 |
| | | E.0 | E.0 | E.0 | E.0 |
| | | F.0 | F.0 | F.0 | F.0 |
| | Day 21 - Lethargic for 1 week after Adria #2, decreased appetite | | | | |

**Criteria for assessment of the canine inflammatory bowel disease activity index (CIBDAI):**

| | |
|---|---|
| **A. Attitude/activity** | **D. Stool consistency** |
| 0 = normal | 0 = normal |
| 1 = slightly decreased | 1 = slightly soft faeces or faecal blood, mucus |
| 2 = moderately decreased | or both |
| 3 = severely decreased | 2 = very soft faeces |
| | 3 = watery diarrhea |
| **B. Appetite** | |
| 0 = normal | **E. Stool frequency** |
| 1 = slightly decreased | 0 = normal |
| 2 = moderately decreased | 1 = slightly increased (2-3 times/day) |
| 3 = severely decreased | 2 = moderately increased (4-5 times/day) |
| | 3 = severely increased (>5 times/day) |
| **C. Vomiting** | |
| 0 = none | **F. Weight loss** |
| 1 = mild (1 episode/week) | 0 = none |
| 2 = moderate (2-3 episodes/week) | 1 = mild (<5% loss) |
| 3 = severe (>3 episodes/week) | 2 = moderate (5-10% loss) |
| | 3 = severe (>10% loss) |
| | |
| | **CIBDAI Scores:** |
| | 0-3 = clinically insignificant disease |
| | 4-5 = mild IBD |
| | 6-8 = moderate IBD |
| | 9 or greater = severe IBD |

### EXAMPLE 9

32 dogs presented with diarrhea following the administration of a chemotherapy agent such as doxorubicin, cytoxan, vincristine, lomustine, and others for a minimum period of 48 hours. CASAD in 500 mg tablets was given orally to the cancer-treated dogs four times a day (*q.i.d.*). Other pharmaceutical agents or medications, as shown below, were also administered to certain dogs according to Best Clinical Practices, using usual, customary dosing. To the extent permitted by scheduling, CASAD and the other medication were given concurrently. After treatment with CASAD, the clinical symptoms of diarrhea resolved within 24-72 hours in 25 dogs. No negative interactions (anorexia, vomiting, nausea, lethargy, anaphylaxis) with any additional drugs were noted in any of the 32 dogs. Doxycycline is a tetracycline antibiotic; Clavamox is an penicillin antibiotic; flagyl is metranidazole, a colonic antiinflammatory antibiotic; Imodium® is an weak opioid; prednisone is a steroid. The results are shown in Table 3 below.

**Table 3**

| Dog # | CASAD | doxycycline | clivamox | flagyl | Imodium ® | prednisone | 1=resolved | 1=interaction |
|---|---|---|---|---|---|---|---|---|
| | | | | | | | 0=nonresolved | 0=noninteraction |
| 1 | 1 | | | 1 | | 1 | 1 | 0 |
| 2 | 1 | | | 1 | | | 1 | 0 |
| 3 | 1 | | | 1 | | 1 | 1 | 0 |
| 4 | 1 | | | | | 1 | 1 | 0 |
| 5 | 1 | | | | | 1 | 1 | 0 |
| 6 | 1 | | | 1 | | | 1 | 0 |
| 7 | 1 | | | 1 | | 1 | 1 | 0 |
| 8 | 1 | | 1 | | | 1 | 1 | 0 |
| 9 | 1 | | | 1 | | | 1 | 0 |
| 10 | 1 | | | 1 | | | 1 | 0 |
| 11 | 1 | 1 | | 1 | | 1 | 1 | 0 |
| 12 | 1 | | | | | 1 | 1 | 0 |
| 13 | 1 | | | 1 | | 1 | 1 | 0 |
| 14 | 1 | | | 1 | | | 1 | 0 |
| 15 | 1 | | | 1 | | 1 | 1 | 0 |
| 16 | 1 | 1 | | | | 1 | 1 | 0 |
| 17 | 1 | 1 | | 1 | | | 1 | 0 |
| 18 | 1 | | | | | 1 | 1 | 0 |
| 19 | 1 | | | | | | 1 | 0 |
| 20 | 1 | | | 1 | | | 1 | 0 |
| 21 | 1 | | | 1 | 1 | | 1 | 0 |
| 22 | 1 | | | 1 | | 1 | 1 | 0 |
| 23 | 1 | | | | | 1 | 1 | 0 |
| 24 | 1 | | | 1 | | | 1 | 0 |
| 25 | 1 | | | 1 | | 1 | 1 | 0 |
| 26 | 1 | 1 | | | | 1 | 0 | 0 |
| 27 | 1 | | | 1 | | | 0 | 0 |
| 28 | 1 | | | 1 | | 1 | 0 | 0 |
| 29 | 1 | 1 | | 1 | | 1 | 0 | 0 |
| 30 | 1 | | | | | 1 | 0 | 0 |
| 31 | 1 | | | | | 1 | 0 | 0 |
| 32 | 1 | | | | | 1 | 0 | 0 |
| | 32 | 5 | 2 | 20 | 1 | 21 | 25 | 0 |

### EXAMPLE 10

The amount of dioxin present in CASAD clay containing a variety of particle sizes and the amount of dioxin present in CASAD clay after being sized to contain only particles less than 80 microns was measured using GC and a mass spectrometer. Prior to sizing, the CASAD clay contained the amounts of dioxin shown in Table 4 below.

**Table 4**

| Analyte | Concentration Found (pg/L) | Detection Limit (pg/L) |
|---|---|---|
| 2,3,7,8-TCDD | -- | 0.024 |
| 1,2,3,7,8-PeCDD | -- | 0.025 |
| 1,2,3,4,7,8-HxCDD | -- | 0.039 |
| 1,2,3,6,7,8-HxCDD | -- | 0.044 |
| 1,2,3,7,8,9-HxCDD | -- | 0.042 |
| 1,2,3,4,6,7,8-HpCDD | 0.121 | 0.043 |
| OCDD | 1.243 | 0.108 |
| Total Tetra-Dioxins | 1.284 | 0.024 |
| Total Penta-Dioxins | 1.820 | 0.025 |
| Total Hexa-Dioxins | 1.994 | 0.039 |
| Total Hepta-Dioxins | -- | 0.043 |

As shown in Table 4, CASAD clay prior to sizing contained 0.121 pg/L of hepta-chlorinated dioxin (1,2,3,4,6,7,8-HpCDD) and 1.243 pg/L of octa-chlorinated dioxin (OCDD). In addition, the total tetra-dioxins were measured at 1.284 pg/L, the total penta-dioxins were measured at 1.820, and the total hexa-dioxins were measured at 1.994. The other dioxins tested were either absent or at a level below the detection limit of the testing apparatus. The CASAD clay was then sized so that it contained only particles less than 80 microns in size. The same analysis of dioxin content was performed. The results are shown in Table 5 below.

**Table 5**

| Analyte | Concentration Found (pg/L) | Detection Limit (pg/L) |
|---|---|---|
| 2,3,7,8-TCDD | -- | 0.024 |
| 1,2,3,7,8-PeCDD | -- | 0.025 |
| 1,2,3,4,7,8-HxCDD | -- | 0.039 |
| 1,2,3,6,7,8-HxCDD | -- | 0.044 |
| 1,2,3,7,8,9-HxCDD | -- | 0.042 |
| 1,2,3,4,6,7,8-HpCDD | -- | 0.043 |
| OCDD | 0.362 | 0.108 |
| Total Tetra-Dioxins | -- | 0.024 |
| Total Penta-Dioxins | -- | 0.025 |
| Total Hexa-Dioxins | -- | 0.039 |
| Total Hepta-Dioxins | -- | 0.043 |

The results show that dioxin content is greatly reduced in CASAD clay having a particle size less than 80 microns. The only remaining detected dioxin was octa-chlorinated dioxin (OCDD), at a reduced amount of 0.362 pg/L. Thus, the clay of this invention is of lower toxicity than other clays.

One skilled in the art readily appreciates that this invention is well adapted to carry out the objectives and obtain the ends and advantages mentioned as well as those inherent therein. Thus, it should be evident that a composition of CASAD in a capsule form and a tablet form are different, and these different forms of oral dosages, as well as any other dosage forms, can be used a method to prevent or treat environmental toxin poisoning, related liver cancer, or the symptoms of diarrhea. Additionally, variations of the composition and methods are encompassed by the invention. For example, techniques may change as manufacturing of larger quantities of the composition are needed. The materials, methods, procedures and techniques described herein are presently representative of the preferred embodiments and are intended to be exemplary. It is understood that one of ordinary skill in the art of pharmaceutical sciences would have available many pharmaceutical reference books, such as Remmington's Pharmaceutical Sciences 17th Edition. Alfonso Gennaro editor, Mack Publishing Company Easton, Pennsylvania 18042, that would allow one to modify and change formulations for the compositions and method of this invention.

### REFERENCES CITED

### U.S. PATENT DOCUMENTS

United States Patent 5,178,832, issued to Phillips, et al., on January 12, 1993, and titled "Selective Immobilization and Detection of Mycotoxins in Solution."
United States Patent 5,165,946 issued to Taylor, et al., on November 24, 1992, titled "Animal Feed Additive and Method for Inactivating Mycotoxins Present in Animal Feeds."

### OTHER PUBLICATIONS

Remmington's Pharmaceutical Sciences 17th Edition. Alfonso Gennaro editor, Mack Publishing Company Easton, Pennsylvania 18042, Entire Book, pages 1-1983.
Remmington's Pharmaceutical Sciences 17th Edition. Alfonso Gennaro editor, Mack Publishing Company Easton, Pennsylvania 18042, Chapter 68, pages 1278-1321.
Remmington's Pharmaceutical Sciences 17th Edition. Alfonso Gennaro editor, Mack Publishing Company, Easton, Pennsylvania 18042, Chapter 84, pages 1492-1517.
Yao-Zong Y, Shi-Rong L, Delvaux. Comparative efficacy of diactahedral smectite (Smecta) and a probiotic preparation in chronic functional diarrhea. Dig Liver Dis. 2004;36(12);824-848.
Gonzalez R, de Medina FS, Martinez- Augustin O, et al. Anti-inflammatory effect of diosmectite in hapten-induced colitis in the rat. British Journal of Pharmacology 2004; 141:951-960.
Rateau JG, Morgant G, Droy-Proit MT, et al. A histological, enzymatic and water-electrolyte study of the action of smectite, a mucoprotective clay, on experimental infectious diarrhoae in the rabbit. Current Medical Research and Opinions. 1982;8(4):233-241.
Narkeviciute I, Rudzeviciene O, Leviniene G, et al. Management of Lithuanian Children's Acute Diarrhoea with Gastrolit Solution and Dioctahedral Smectite. European Journal of Gastroenterology and Hepatology. 2000;4:419-424.
Madkour AA, Madina EM, El-Azzouni, et al. Smectite in Acute Infectious Diarrhea in Children: A double blinded Placebo Controlled Clinical Trial. Journal of Pediatric Gastroenterology and Nutrition. 1993;17:176-181.
Samuel MJ. Paediatric Forum: Acute Diarrhea. African Health 1995;17(2):27, 29-30.
Szajeska H, Dziechciarz P, Mrukowicz J. Meta-Analysis: Smectite in the treatment of acute infectious diarrhea in children. Aliment Pharmacol Ther. 2006;23(2):217-227.
Yen ZS, Lai MS. Best Evidence Topic Report. Smectite for Acute Diarrhoea in Children. Emerg Med Journal. 2006;23(1):65-66.
Desjeux JF, Mary JY, Flori YA. Agents for diarrhoea in children. The Lancet 1991;337:924-925.
Weber W. A new suspension form of smectite (Liquid 'Diasob') for the treatment of acute diarrhea: a randomized comparative study. Pharmatherapeutica. 1988;5(2):256-260.
Hombrink J, Frohlich D, Glatzel M, et al. Prevention of radiation-induced diarrhea by smectite. Results of a double blinded randomized, placebo-controlled multicenter study. Strahlenther Onkol. 2000;176(4):173-179.
Ogilvie GK, et al., "Acute and Short-term Toxicoses Associated with the Administration of Doxorubicin to Dogs with Malignant Tumors." Am Vet Med Assoc. 1989 Dec 1;195(11):1584-7.
Wang J-S, Luo H, Billam M, Wang Z, Guan H, Tang L, Goldston T, Afriyie-Gyawu E, Lovett C, Griswold J, et al. 2005. Short-term safety evaluation of processed calcium montmorillonite clay (NovaSil) in humans. Food Additives and Contaminants 22:270-279.

## Claims

1. An oral composition for use as a supportive therapy for treating diarrhea in a subject comprising:
an effective amount of an isolated low sodium calcium aluminosilicate anti-diarrheal ("CASAD"), wherein the isolated low sodium CASAD has an average particle size between 5 and 100 microns, is substantially free from dioxins and toxic heavy metal contamination and has a chemical composition comprising CaO above about 3.2%; MgO ranging from 4.0 to 5.4%; Fe₂O₃ ranging from 5.4 to 6.5%; K₂O ranging from 0.50 to 0.90%; Na₂O ranging from 0.10 to 0.30%; MnO ranging from 0.01 to 0.03%; Al₂O₃ ranging from 14.8 to 18.2%; and SiO₂ ranging from 62.4 to 73.5%; wherein, the chemical composition is given as weight percent.

2. The composition for use according to claim 1, wherein the diarrhea is associated with one or more treatment condition or disease selected from the group comprising;
(a) an infectious or chronic disease in the subject,
(b) a disease or condition causing inflammation through action of inflammatory proteins in the subject, and the isolated CASAD is capable of binding the inflammatory proteins,
(c) treatment using a drug that is metabolized into drug metabolites in the subject, and the isolated CASAD is capable of binding the drug metabolites;
(d) treatment using a chemotherapeutic agent in the subject, and the isolated CASAD is capable of binding the chemotherapeutic agent,
(e) treatment with doxorubicin, or
(f) treatment using radiation in the subject.

3. The composition for use according to claim 1 or 2, wherein the isolated CASAD has an average particle size that is between between 5 and 50 microns.

4. The composition for use according to any one of the preceding claims wherein the isolated CASAD has a pH in the range of about 5 to 9 in a suspension.

5. The composition for use according to any one of the preceding claims, wherein the isolated CASAD is in tablet, powder, capsule, or suspension form.

6. The composition for use according to any one of the preceding claims, further comprising one or more pharmaceutical agents or pharmaceutically acceptable carriers.

7. The composition for use according to any one of the preceding claims, wherein the one or more pharmaceutical agents comprise one or more antibiotics, chemotherapeutic agents, anti-diarrhea agents, steroids, opioids, or gastric antacids.

8. Use of an effective amount of an isolated low sodium calcium aluminosilicate anti-diarrheal ("CASAD") clay, wherein the isolated CASAD is substantially free from dioxin and toxic metal contamination, in the preparation of a medicament for supportive therapy of symptoms of diarrhea, wherein the isolated low sodium CASAD has an average particle size between 5 and 100 microns and has a chemical composition comprising CaO above about 3.2%; MgO ranging from 4.0 to 5.4%; Fe₂O₃ ranging from 5.4 to 6.5%; K₂O ranging from 0.50 to 0.90%; Na₂O ranging from 0.10 to 0.30%; MnO ranging from 0.01 to 0.03%; Al₂O₃ ranging from 14.8 to 18.2%; and SiO₂ ranging from 62.4 to 73.5%; wherein, the chemical composition is given as weight percent.

## Patentansprüche

1. Eine orale Zusammensetzung zur Verwendung als unterstützende Therapie zur Behandlung von Diarrhoe in einem Subjekt, umfassend:
eine wirksame Menge eines isolierten natriumarmen Calcium-Aluminosilikat-Antidiarrhoikums ("CASAD"), wobei das isolierte natriumarme CASAD eine mittlere Teilchengröße zwischen 5 und 100 Mikrometer aufweist, im Wesentlichen frei von Dioxinen und toxischer Schwermetallkontamination ist und eine chemische Zusammensetzung aufweist, die CaO oberhalb von etwa 3,2%; MgO im Bereich von 4,0 bis 5,4%; Fe₂O₃ im Bereich von 5,4 bis 6,5%; K₂O im Bereich von 0,50 bis 0,90%; Na₂O im Bereich von 0,10 bis 0,30%; MnO im Bereich von 0,01 bis 0,03%; Al₂O₃ im Bereich von 14,8 bis 18,2%; und SiO₂ im Bereich von 62,4 bis 73,5% umfasst; wobei die chemische Zusammensetzung in Gewichtsprozent angegeben ist.

2. Die Zusammensetzung zur Verwendung gemäß Anspruch 1, wobei die Diarrhoe mit einer oder mehreren Behandlungsbedingungen oder Erkrankungen verbunden ist, die ausgewählt sind aus der Gruppe bestehend aus;
(a) einer infektiösen oder chronischen Erkrankung in dem Subjekt,
(b) einer Erkrankung oder einem Zustand, die(der) eine Entzündung durch die Wirkung von entzündlichen Proteinen in dem Subjekt verursacht und wobei das isolierte CASAD dazu in der Lage ist, die entzündlichen Proteine zu binden,
(c) der Behandlung unter Verwendung eines Arzneimittels, welches in dem Subjekt in Arzneimittelmetaboliten metabolisiert ist und wobei das isolierte CASAD dazu in der Lage ist, die Arzneimittelmetabolite zu binden,
(d) der Behandlung unter Verwendung eines chemotherapeutischen Wirkstoffs in dem Subjekt und wobei das isolierte CASAD dazu in der Lage ist, den chemotherapeutische Wirkstoff zu binden,
(e) der Behandlung mit Doxorubicin oder
(f) der Behandlung unter Verwendung von Strahlung in dem Subjekt.

3. Die Zusammensetzung zur Verwendung gemäß Anspruch 1 oder 2, wobei das isolierte CASAD eine mittlere Teilchengröße aufweist, die zwischen 5 und 50 Mikrometer liegt.

4. Die Zusammensetzung zur Verwendung gemäß einem der vorstehenden Ansprüche, wobei das isolierte CASAD einen pH-Wert im Bereich von etwa 5 bis 9 in einer Suspension aufweist.

5. Die Zusammensetzung zur Verwendung gemäß einem der vorstehenden Ansprüche, wobei das isolierte CASAD in der Form einer Tablette, Pulver, Kapsel oder Suspension vorliegt.

6. Die Zusammensetzung zur Verwendung gemäß einem der vorstehenden Ansprüche, ferner umfassend einen oder mehrere Wirkstoffe oder pharmazeutisch verträgliche Trägerstoffe.

7. Die Zusammensetzung zur Verwendung gemäß einem der vorstehenden Ansprüche, wobei der eine oder die mehreren Wirkstoffe einen oder mehrere Antibiotika, chemotherapeutische Wirkstoffe, Antidiarrhoika, Steroide, Opioide oder gastrische Antazida umfassen.

8. Verwendung einer wirksamen Menge eines isolierten natriumarmen Calcium-Aluminosilikat-Antidiarrhoikum ("CASAD")-Tons, wobei das isolierte CASAD im Wesentlichen frei von Dioxin und toxischer Metallkontamination ist, zur Herstellung eines Medikaments zur unterstützenden Therapie von Symptomen von Diarrhoe, wobei das isolierte natriumarme CASAD eine mittlere Teilchengröße zwischen 5 und 100 Mikrometer aufweist und eine chemische Zusammensetzung aufweist, die CaO oberhalb von etwa 3,2%; MgO im Bereich von 4,0 bis 5,4%; Fe₂O₃ im Bereich von 5,4 bis 6,5%; K₂O im Bereich von 0,50 bis 0,90%; Na₂O im Bereich von 0,10 bis 0,30%; MnO im Bereich von 0,01 bis 0,03%; Al₂O₃ im Bereich von 14,8 bis 18,2%; und SiO₂ im Bereich von 62,4 bis 73,5% umfasst; wobei die chemische Zusammensetzung in Gewichtsprozent angegeben ist.

## Revendications

1. Composition orale pour utilisation en tant que traitement de soutien pour traiter la diarrhée chez un sujet comprenant :
une quantité efficace d'un aluminosilicate de calcium anti-diarrhéique (« CASAD ») isolé pauvre en sodium, dans laquelle le CASAD isolé pauvre en sodium a une taille moyenne de particule entre 5 et 100 microns, est sensiblement exempt de dioxines et de contamination par des métaux lourds toxiques et a une composition chimique comprenant du CaO au-dessus d'environ 3,2 % ; du MgO se situant dans la plage de 4,0 à 5,4 % ; du Fe₂O₃ se situant dans la plage de 5,4 à 6,5 % ; du K₂O se situant dans la plage de 0,50 à 0,90 % ; du Na₂O se situant dans la plage de 0,10 à 0,30 % ; du MnO se situant dans la plage de 0,01 à 0,03 % ; de l'Al₂O₃ se situant dans la plage de 14,8 à 18,2 % ; et du SiO₂ se situant dans la plage de 62,4 à 73,5 % ; dans laquelle la composition chimique est donnée en pourcentage en poids.

2. Composition pour utilisation selon la revendication 1, dans laquelle la diarrhée est associée à un(e) ou plusieurs traitement, affection ou maladie choisi(e)s dans le groupe comprenant ;
(a) une maladie infectieuse ou chronique chez le sujet,
(b) une maladie ou une affection provoquant une inflammation due à l'action de protéines inflammatoires chez le sujet, et le CASAD isolé est capable de se lier aux protéines inflammatoires,
(c) un traitement utilisant un médicament qui est métabolisé en métabolites de médicament chez le sujet, et le CASAD isolé est capable de se lier aux métabolites de médicament ;
(d) un traitement utilisant un agent chimiothérapeutique chez le sujet, et le CASAD isolé est capable de se lier à l'agent chimiothérapeutique,
(e) un traitement par de la doxorubicine, ou
(f) un traitement utilisant un rayonnement chez le sujet.

3. Composition pour utilisation selon la revendication 1 ou 2, dans laquelle le CASAD isolé a une taille moyenne de particule qui est entre 5 et 50 microns.

4. Composition pour utilisation selon l'une quelconque des revendications précédentes dans laquelle le CASAD isolé a un pH dans la plage d'environ 5 à 9 dans une suspension.

5. Composition pour utilisation selon l'une quelconque des revendications précédentes, dans laquelle le CASAD isolé est sous la forme d'un comprimé, d'une poudre, d'une capsule ou d'une suspension.

6. Composition pour utilisation selon l'une quelconque des revendications précédentes, comprenant en outre un ou plusieurs agents pharmaceutiques ou vecteurs pharmaceutiquement acceptables.

7. Composition pour utilisation selon l'une quelconque des revendications précédentes, dans laquelle les un ou plusieurs agents pharmaceutiques comprennent un ou plusieurs antibiotiques, agents chimiothérapeutiques, agents anti-diarrhéiques, stéroïdes, opioïdes ou antiacides gastriques.

8. Utilisation d'une quantité efficace d'une argile d'aluminosilicate de calcium anti-diarrhéique (« CASAD ») isolé pauvre en sodium, dans laquelle le CASAD isolé est sensiblement exempt de dioxine et de contamination par des métaux lourds toxiques, dans la préparation d'un médicament pour le traitement de soutien de symptômes de diarrhée, dans laquelle le CASAD isolé pauvre en sodium a une taille moyenne de particule entre 5 et 100 microns et a une composition chimique comprenant du CaO au-dessus d'environ 3,2 % ; du MgO se situant dans la plage de 4,0 à 5,4 % ; du Fe₂O₃ se situant dans la plage de 5,4 à 6,5 % ; du K₂O se situant dans la plage de 0,50 à 0,90 % ; du Na₂O se situant dans la plage de 0,10 à 0,30 % ; du MnO se situant dans la plage de 0,01 à 0,03 % ; de l'Al₂O₃ se situant dans la plage de 14,8 à 18,2 % ; et du SiO₂ se situant dans la plage de 62,4 à 73,5 % ; dans laquelle la composition chimique est donnée en pourcentage en poids.
